(19) Europäisches Patentamt
European Patent Office
Office européen des brevets

(11) Veröffentlichungsnummer : **0 558 443 A1**

(12) # EUROPÄISCHE PATENTANMELDUNG

(21) Anmeldenummer : **93810079.9**

(22) Anmeldetag : **09.02.93**

(51) Int. Cl.[5] : **C07D 211/22,** C07D 211/42, C07D 211/46, A61K 31/445

(30) Priorität : **17.02.92 CH 460/92**

(43) Veröffentlichungstag der Anmeldung : **01.09.93 Patentblatt 93/35**

(84) Benannte Vertragsstaaten : **AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Frei, Jörg, Dr.**
**Buechring 36**
**CH-4434 Hölstein (CH)**
Erfinder : **Stanek, Jaroslav, Dr.**
**Hangstrasse 9**
**CH-4144 Arlesheim (CH)**

(54) **O-Piperidyl-(3/4)-Hydroxylamine und O-piperidyl-(3/4)-alkyl-Hydroxylamine und ihre Verwendung als Ornitin-decarboxylase Inhibitoren.**

(57) Die Erfindung betrifft Verbindungen der Formel I,

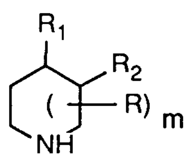

(I)

worin entweder $R_1$ ein Radikal der Formel Ia,

$-(CH_2)_n-O-NH_2$ (Ia)

in dem n 0 oder 1 ist, und $R_2$ Wasserstoff bedeuten, oder $R_1$ Wasserstoff und $R_2$ ein Radikal der Formel Ib,

$-(CH_2)_p-O-NH_2$ (Ib)

in dem p 1 oder 2 ist, bedeuten ; und worin R $C_1$-$C_2$-Alkyl bedeutet, welches an ein Kohlenstoffatom des zentralen Piperidinringsystems gebunden ist, aber nicht an dasselbe Kohlenstoffatom wie $R_1$ der Formel Ia oder wie $R_2$ der Formel Ib ; wobei m 1 oder 2 bedeutet, und Salze davon, Verfahren zur Herstellung dieser Verbindungen, bei der Herstellung erhaltene Zwischenprodukte, pharmazeutische Präparate, die diese Verbindungen enthalten, und die Verwendung dieser Verbindungen zur therapeutischen Behandlung des menschlichen oder tierischen Körpers und zur Herstellung pharmazeutischer Präparate. Die Verbindungen der Formel I sind Ornithindecarboxylase-Hemmer.

**EP 0 558 443 A1**

Jouve, 18, rue Saint-Denis, 75001 PARIS

Die Erfindung betrifft Verbindungen der Formel I,

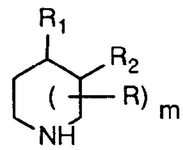

(I)

worin entweder $R_1$ ein Radikal der Formel Ia,

$-(CH_2)_2-O-NH_2$ (Ia)

in dem n 0 oder 1 ist, und $R_2$ Wasserstoff bedeuten, oder $R_1$ Wasserstoff und $R_2$ ein Radikal der Formel Ib,

$-(CH_2)_p-O-NH_2$ (Ib)

in dem p 1 oder 2 ist, bedeuten; und worin R $C_1$-$C_2$-Alkyl bedeutet, welches an ein Kohlenstoffatom des zentralen Piperidinringsystems gebunden ist, aber nicht an dasselbe Kohlenstoffatom wie $R_1$ der Formel Ia oder wie $R_2$ der Formel Ib; wobei m 1 oder 2 bedeutet, und Salze davon, Verfahren zur Herstellung dieser Verbindungen, bei der Herstellung erhaltene Zwischenprodukte, pharmazeutische Präparate, die diese Verbindungen enthalten, und die Verwendung dieser Verbindungen zur therapeutischen Behandlung des menschlichen oder tierischen Körpers und zur Herstellung pharmazeutischer Präparate.

Die Substituenten $R_1$ oder $R_2$ sowie die Substituenten R können auf der gleichen Seite oder unabhängig voneinander auf verschiedenen Seiten in Bezug auf die Ebene des zentralen Piperidinringsystems der Verbindungen der Formel I angeordnet sein, so dass verschiedene Isomeren (Diastereomere, Enantiomere) oder Isomerengemische denkbar sind. Vorzugsweise stehen alle vorhandenen Substituenten R auf einer Seite in Bezug auf die Ringebene, während der Rest $R_1$ oder $R_2$ in trans- oder in cis-Stellung zu den Resten R steht, besonders bevorzugt in trans-Stellung.

Bei Vorliegen von Asymmetriezentren können diese unabhängig voneinander in der R-, der S- oder der R,S-Konfiguration vorliegen. Vorzugsweise können die Verbindungen der vorliegenden Erfindung, die über mindestens ein asymmetrisches Kohlenstoffatom verfügen, in Form von reinen Enantiomeren oder als Enantiomerengemische (Racemate) vorliegen.

Die in der Beschreibung der vorliegenden Erfindung verwendeten Bezeichnungen und allgemeinen Ausdrücke haben vorzugsweise die folgenden Bedeutungen:

Wenn $R_1$ ein Radikal der Formel Ia bedeutet, ist n 0 oder 1, vorzugsweise 0.

Wenn $R_2$ ein Radikal der Formel Ib bedeutet, ist p 1 oder 2, vorzugsweise 1.

Wenn m 2 ist, können die Reste R unabhängig voneinander $C_1$-$C_2$-Alkyl, z. B. Methyl oder Ethyl, bedeuten. Bevorzugt sind dann solche Verbindungen, in denen alle vorliegenden Reste R gleich (entweder alle Methyl oder alle Ethyl) sind.

Ein Rest R ist nicht an dasjenige Kohlenstoffatom gebunden, an das entweder ein Rest $R_1$ der Formel Ia (d. h., $R_1$ ist kein Wasserstoff) oder ein Rest $R_2$ der Formel Ib (d. h., $R_2$ ist kein Wasserstoff) gebunden ist.

Salze von erfindungsgemässen Verbindungen sind in erster Linie pharmazeutisch annehmbare Säureadditionssalze, d. h. solche Säureadditionssalze, die in den jeweils anzuwendenden Dosen keine nennenswerte Toxizität aufweisen, z.B. mit anorganischen Säuren, wie Salzsäure, Schwefelsäure oder Phosphorsäure, oder mit geeigneten organischen Carbon- oder Sulfonsäuren, z.B. Essigsäure, Octansäure, Bernsteinsäure, Adipinsäure, Fumarsäure, Maleinsäure, Hydroxymaleinsäure, Propionsäure, Milchsäure, Aepfelsäure, Zitronensäure, Salicylsäure, p-Aminosalicylsäure, Ascorbinsäure, Oxalsäure, Benzolsulfonsäure, 1,5-Naphthalindisulfonsäure, Methansulfonsäure oder 1,2-Ethandisulfonsäure, mit N-Cyclohexylsulfaminsäure, oder z.B. mit Aminosäuren, wie Glutaminsäure oder Asparaginsäure. Es können Mono- oder Disalze gebildet werden, je nach Anzahl und Basizität der vorhandenen basischen Gruppen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch annehmbaren Salze, die deshalb bevorzugt sind.

Die erfindungsgemässen Verbindungen weisen wertvolle, insbesondere pharmakologisch verwendbare, Eigenschaften auf. Überraschenderweise wurde gefunden, dass die Verbindungen der Formel I insbesondere starke, spezifische Hemmwirkung auf das Enzym Ornithindecarboxylase (ODC) haben. Sie stellen eine neue Klasse von ODC-Hemmern dar.

ODC spielt als ein Schlüsselenzym eine wichtige Rolle bei der Polyaminbiosynthese, die in praktisch allen Zellen von Säugetieren, einschliesslich Menschen, abläuft. Durch ODC wird die Polyaminkonzentration in der Zelle reguliert. Eine Hemmung des Enzyms ODC hat eine Verringerung der Polyaminkonzentration zur Folge. Da eine Verringerung der Polyaminkonzentration eine Hemmung des Zellwachstums bewirkt, ist es möglich, durch Verabreichung von ODC-hemmenden Substanzen das Wachstum von eukaryotischen wie auch von prokaryotischen, inbesondere von rasch oder unkontrollierbar wachsenden, Zellen zu hemmen und sogar Zellen abzutöten oder das Einsetzen der Zelldifferenzierung zu hemmen.

Die Hemmung des Enzyms ODC kann z.B. mit der Methode von J.E. Seely und A.E. Pegg, Ornithin Decarboxylase (Mouse Kidney), Seiten 158-161, in H. Tabor und C. White-Tabor (Hrsg.): Methods in Enzymology, Vol. 94: Polyamines, Academic Press, New York 1983, nachgewiesen werden. Wenn man in diesem Test ODC aus Rattenleber verwendet (Isolierung: Hayashi, S.-I. und Kameji, T., selber Band, Seiten 154-158), werden für die Verbindungen der Formel I $IC_{50}$-Werte im mikromolaren Bereich, herunter bis zu etwa 0,2 μM, vorzugsweise zwischen etwa 0,2 und 20 μM, z. B. zwischen 0,21 und 6,1 μM, erhalten. $IC_{50}$ ist die Konzentration des Inhibitors, bei der die ODC-Aktivität 50 % einer Kontrolle ohne Inhibitor beträgt.

Als ODC-Hemmer besitzen die Verbindungen der Formel I antiproliferative Eigenschaften, die sich z.B. durch Nachweis der Hemmwirkung auf das Wachstum von menschlichen T24 Blasenkarzinomzellen demonstrieren lassen. Der Nachweis erfolgt, indem man diese Zellen in "Eagle's minimal essential medium", dem 5 % (V/V) fötales Kälberserum zugesetzt werden, in einem befeuchteten Inkubator bei 37°C und 5 Volumenprozent $CO_2$ in der Luft inkubiert. Die Karzinomzellen (1000-1500) werden in 96-Loch-Mikrotiterplatten überimpft und über Nacht unter den genannten Bedingungen inkubiert. Die Testsubstanz wird in seriellen Verdünnungen am Tag 1 hinzugefügt. Die Platten werden unter den genannten Bedingungen 5 Tage lang inkubiert. Während dieser Zeitspanne durchlaufen die Kontrollkulturen mindestens 4 Zellteilungen. Nach der Inkubation werden die Zellen mit 3,3%iger (Gewicht/Volumen = G/V) wässriger Glutaraldehydlösung fixiert, mit Wasser gewaschen und mit 0,05%iger (G/V) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportinal ist, mit einem Photometer (Titertek multiskan) bei 665 nm gemessen. Die $IC_{50}$-Werte werden mit einem Computersystem unter Verwendung der Formel

$$IC_{50} = \frac{OD_{665}\,(\text{Test}) - OD_{665}\,(\text{Anfang})}{OD_{665}\,(\text{Kontrolle}) - OD_{665}\,(\text{Anfang})} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt.

Die Verbindungen der Formel I sind also insbesondere geeignet zur Behandlung von Krankeitszuständen, die auf eine Hemmung der Ornithindecarboxylase ansprechen, z. B. benigner und maligner Tumoren. Sie können Tumorregressionen bewirken und ferner die Verbreitung von Tumorzellen sowie das Wachstum von Mikrometastasen verhindern. Des weiteren können sie z.B. zur Behandlung von Protozoainfektionen, wie etwa Trypanosomiasis, Malaria oder durch Pneumocystis carinii verursachte Lungenentzündung, dienen.

Als selektive ODC-Hemmer können die Verbindungen der Formel I allein oder auch in Kombination mit anderen pharmakologisch wirksamen Substanzen angewendet werden. Zu denken ist z. B. an eine Kombination mit (a) Inhibitoren anderer Enzyme der Polyaminbiosynthese, z. B. S-Adenosylmethionindecarboxylasehemmern, (b) Inhibitoren der Proteinkinase C, (c) Inhibitoren der Tyrosinproteinkinase, (d) Cytokinen, (e) negativen Wachstumsregulatoren, (f) Aromatasehemmern, (g) Antiöstrogenen oder (h) klassischen zytostatischen Wirkstoffen.

Bevorzugt betrifft die Erfindung Verbindungen der Formel I, worin R nur an solchen Ringkohlenstoff gebunden ist, der an das Stickstoffheteroatom des zentralen Piperidinringsystems direkt gebunden ist, und die übrigen Symbole die oben genannten Bedeutungen haben, und Salze davon.

Bevorzugt sind auch Verbindungen der Formel I, worin im Falle der Definition des Restes $R_1$ durch das Radikal der Formel Ia n 0 ist oder im Falle der Definition des Restes $R_2$ durch das Radikal der Formel Ib p 1 ist, und die übrigen Symbole die oben genannten Bedeutungen haben, und Salze davon.

Sehr bevorzugt sind Verbindungen der Formel I, worin im Falle der Definition des Restes $R_1$ durch das Radikal der Formel Ia n 0 ist oder im Falle der Definition des Restes $R_2$ durch das Radikal der Formel Ib p 1 ist, und die übrigen Symbole die oben genannten Bedeutungen haben, wobei R nur an solchen Ringkohlenstoff gebunden ist, der an das Stickstoffheteroatom des zentralen Piperidinringsystems direkt gebunden ist, und, insbesondere pharmazeutisch annehmbare, Salze davon.

Besonders hervorzuheben sind Verbindungen der Formel II,

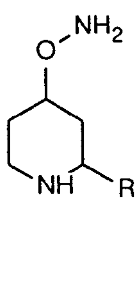

(II)

.

worin R $C_1$-$C_2$-Alkyl bedeutet, und pharmazeutisch annehmbare Salze davon.

Hierunter sind die Verbindungen der Formel II, worin der Rest -O-$NH_2$ und der Rest R, welcher $C_1$-$C_2$-Alkyl, d. h. Methyl oder Ethyl bedeutet, in trans-Stellung zueinander an das zentrale Piperidinringsystem gebunden sind, und pharmazeutisch annehmbare Salze davon, sehr hervorzuheben.

In erster Linie bevorzugt ist die Verbindung der Formel II, worin R Methyl bedeutet und in trans-Stellung zum Rest -O-$NH_2$ an das zentrale Piperidinringsystem gebunden ist, oder ein pharmazeutisch annehmbares Salz davon.

Besonders hervorzuheben sind auch Verbindungen der Formel III,

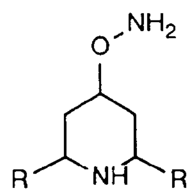

(III)

worin die Reste R unabhängig voneinander $C_1$-$C_2$-Alkyl bedeuten, und pharmazeutisch annehmbare Salze davon.

In erster Linie bevorzugt ist eine Verbindung der Formel III, worin R Methyl bedeutet und die beiden Reste R in cis-Stellung zueinander, jedoch in trans-Stellung zum Rest -O-$NH_2$ an das zentrale Piperidinringsystem gebunden sind, oder ein pharmazeutisch annehmbares Salz davon.

Die Erfindung betrifft vor allem die in den Beispielen beschriebenen spezifischen Verbindungen und pharmazeutisch annehmbare Salze davon.

Die neuen Verbindungen und ihre Salze können nach an sich bekannten Verfahren hergestellt werden, indem man z. B.

(a) aus einer Verbindung der Formel I, worin mindestens eine Aminogruppe geschützt ist, vorzugsweise einer Verbindung der Formel IV

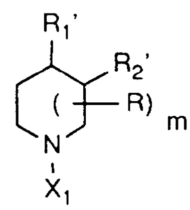

(IV)

oder einem Salz davon, sofern salzbildende Gruppen vorliegen, worin entweder $R_1$' ein Radikal der Formel IVa,

$$-(CH_2)_n-O-NX_2X_3 \qquad (IVa)$$

worin n 0 oder 1 ist, und $R_2$' Wasserstoff bedeuten, oder $R_1$' Wasserstoff und $R_2$' ein Radikal der Formel IVb,

$$-(CH_2)_p-O-NX_2X_3 \qquad (IVb)$$

worin p 1 oder 2 ist, bedeuten, und die übrigen Symbole die für Verbindungen der Formel I genannten Bedeutungen haben, wobei $X_1$, $X_2$ und $X_3$ unabhängig voneinander für eine Aminoschutzgruppe oder Wasserstoff stehen, mit der Massgabe, dass mindestens eine der Gruppen $X_1$, $X_2$ und $X_3$ eine Aminoschutzgruppe bedeutet, oder worin $X_1$ für eine Aminoschutzgruppe oder Wasserstoff steht und $X_2$ zusammen mit $X_3$ eine bivalente Aminoschutzgruppe bildet, die Aminoschutzgruppe(n) abspaltet, oder

(b) eine Verbindung der Formel V

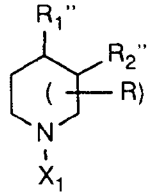 m (V)

oder einem Salz davon, sofern salzbildende Gruppen vorliegen, worin $X_1$ Wasserstoff oder eine Aminoschutzgruppe bedeutet; entweder $R_1$" einen Rest der Formel Va,

$$-(CH_2)_n-W_1 \qquad (Va)$$

worin $W_1$ eine Abgangsgruppe bedeutet und n 0 oder 1 ist, und $R_2$" Wasserstoff bedeuten; oder $R_1$" Wasserstoff und $R_2$" einen Rest der Formel Vb

$$-(CH_2)_p-W_2 \qquad (Vb)$$

bedeuten, worin W2 eine Abgangsgruppe bedeutet und p 1 oder 2 ist; und die übrigen Symbole die für Verbindungen der Formel I genannten Bedeutungen haben; mit einem aminogeschützten Hydroxylamin-Derivat unter Substitution von entweder $W_1$ oder $W_2$ umsetzt, wobei weitere funktionelle Gruppen in den Ausgangsmaterialien, die nicht an der Reaktion teilnehmen sollen, in geschützter Form vorliegen, und vorhandene Schutzgruppen abspaltet,
und gewünschtenfalls eine Verbindung der Formel I in eine andere Verbindung der Formel I überführt, ein erhältliches Isomerengemisch in die Isomeren aufspaltet und/oder eine erhältliche freie Verbindung der Formel I in ein Salz überführt oder ein erhältliches Salz in die freie Verbindung der Formel I überführt oder in ein anderes Salz umwandelt.

Das nachstehend verwendete Präfix "Nieder" bezeichnet im Rahmen der vorliegenden Anmeldung einen Rest mit 1 bis 7 und insbesondere mit 1 bis 4 Kohlenstoffatomen. Niederalkyl ist z.B. Methyl, Ethyl, Propyl, Butyl, Pentyl, Hexyl, Heptyl, wobei ein Alkylrest mit 3 bis 7 Kohlenstoffatomen geradkettig oder verzweigt, beispielsweise als Isopropyl, Isobutyl, sec-Butyl, tert-Butyl, Isopentyl oder Neopentyl, vorliegen kann.

Alkyl bezeichnet einen gesättigten, verzweigten oder unverzweigten Kohlenwasserstoffrest mit bis zu 20, vorzugsweise bis zu 12 Kohlenstoffatomen und ist beispielsweise Dodecyl, Undecyl, Decyl, Nonyl, Octyl oder insbesondere Niederalkyl.

Aryl hat vorzugsweise bis zu 14 Kohlenstoffatome und ist insbesondere unsubstituiertes oder, z.B. durch Niederalkyl, insbesondere Methyl oder tert-Niederalkyl, wie tert-Butyl, Niederalkoxy, wie Methoxy, Hydroxy, Halogen, z.B. Fluor, Chlor, Brom oder Iod und/oder Nitro, mono- oder poly-, insbesondere mono-, di- oder trisubstituiertes Phenyl, oder Naphthyl oder 9-Fluorenyl, in erster Linie unsubstituiertes oder substituiertes Phenyl.

Verfahren (a):

Bevorzugte monovalente Aminoschutzgruppen $X_1$, $X_2$ und $X_3$ sind Acylgruppen, vorzugsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor oder 2,2,2-Trichloracetyl, unsubstituiertes oder, z.B. durch Halogen, Niederalkoxy, Niederalkoxycarbonyl oder Nitro, substituiertes Benzoyl, z.B. Benzoyl, 4-Chlorbenzoyl, 4-Methoxybenzoyl, 2-Methoxycarbonyl-benzoyl oder 4-Nitrobenzoyl, der Acylrest eines Kohlensäurehalbesters, in erster Linie Arylmethoxycarbonyl mit einem oder zwei Arylresten, die vorzugsweise unsubstituiertes oder, z.B. durch Niederalkyl, insbesondere tert-Niederalkyl, wie tert-Butyl, Niederalkoxy, wie Methoxy, Hydroxy, Halogen, z.B. Chlor und/oder Nitro, mono- oder polysubstituiertes Phenyl, oder Naphthyl oder 9-Fluorenyl darstellen, wie unsubstituiertes oder substituiertes Benzyloxycarbonyl, z.B. 4-Nitrobenzyloxycarbonyl, oder substituiertes Diphenylmethoxycarbonyl, z.B. Di-(4-methoxyphenyl)-methoxycarbonyl, 2-Halogenniederalkoxycarbonyl, z.B. 2,2,2-Trichlorethoxycarbonyl, 2-Brommethoxycarbonyl oder 2-Iodethoxycarbonyl, Niederalkoxycarbonyl, insbesondere ein in 1-Stellung des Niederalkylrestes verzweigtes oder in 1- oder 2-Stellung geeignet substituiertes Niederalkoxycarbonyl, insbesondere tert-Niederalkoxycarbonyl, z.B. tert-Butoxycarbonyl, Alkylformimidoyl, wie Niederalkyl-formimidoyl, z.B. tert-Butyl-formimidoyl, oder Arylmethylgruppen, wie Mono-, Di- oder insbesondere Triarylmethyl, wobei die Arylreste insbesondere unsubstituierte oder substituierte Phenylreste darstellen, z.B. Benzyl, Diphenylmethyl oder Triphenylmethyl (Trityl).

Besonders bevorzugte monovalente Aminoschutzgruppen $X_1$, $X_2$ und $X_3$ sind Acylreste von

Kohlensäurehalbestern, insbesondere Niederalkoxycarbonyl, z.B. tert-Butoxycarbonyl, unsubstituiertes oder, z.B. wie oben angegeben, substituiertes Benzyloxycarbonyl, z.B. 4-Nitro-benzyloxycarbonyl, Diphenylmethoxycarbonyl oder 2-Halogen-niederalkoxycarbonyl, wie 2,2,2-Trichlorethoxycarbonyl, ferner Trityl, Formyl oder 2-Methoxycarbonyl-benzoyl, oder Niederalkylformimidoylreste, insbesondere solche, deren Niederalkylrest in 1-Stellung ein- oder zweifach verweigt ist, wie tert-Butyl-formimidoyl. Als $X_1$ ist in allererster Linie Niederalkoxycarbonyl, z.B. tert-Butoxycarbonyl, bevorzugt.

Bevorzugte bivalente Aminoschutzgruppen, gebildet aus den Resten $X_2$ und $X_3$, sind mono- oder disubstituierte Methylidengruppen, wie 1-Niederalkoxy (insbesondere Methoxy oder Ethoxy)-niederalkyliden (beispielsweise -ethyliden oder-1-n-butyliden), z.B. $=C(CH_3)(OC_2H_5)$, ferner z.B. $=C(CH_3)_2$ oder =CH-Phenyl, und vorzugsweise über beide Carbonylgruppen gebundene Bisacylreste, insbesondere unsubstituiertes oder, beispielsweise durch dieselben Substituenten, wie oben für substituiertes Benzoyl definiert, subtituiertes Phthalyl, z.B. der Phthalylrest, welcher zusammen mit dem zu schützenden Stickstoffatom ein 1H-Isoindol-1,3(2H)-dionradikal (Phthalimidogruppe) bildet, oder Niederalkyl-dicarbonsäurereste, wie der Bernsteinsäurerest, Niederalkenyldicarbonsäurereste, wie der Maleinsäurerest, oder $C_6$-$C_{12}$-Bicyclodicarbonsäurereste, wie der 5-Norbonen-2,3-dicarbonsäurerest.

Aminoschutzgruppen, ihre Einführung und Abspaltung sind an sich bekannt und z.B. in J.F.W. McOmie, "Protective Groups in Organic Chemistry", Plenum Press, London, New York, 1973, und T.W. Greene, "Protective Groups in Organic Synthesis", Wiley, New York, 1984 beschrieben. Die Einführung von Alkylformimidoylresten ist beispielsweise beschrieben von Meyers, A. et al., in J. Am. Chem. Soc. 106, 3270 (1984); der tert-Butylformimidoylrest wird z.B. eingeführt durch Umsetzung der freien Aminoverbindung mit N,N-Dimethyl-N'-butylformamidin in Gegenwart einer katalytischen Menge von Ammoniumsulfat in Toluol bei Rückflusstemperatur, oder alternativ durch Umsetzung von tert-Butylformamid mit $Et_3O^+ BF_4^-$ in Methylenchlorid bei Raumtemperatur, Zugabe der Aminoverbindung und weitere Umsetzung im Temperaturbereich von Raumtemperatur bis 40 °C.

Die Abspaltung der Aminoschutzgruppe(n) erfolgt, gegebenenfalls stufenweise oder gleichzeitig, je nach Art der Schutzgruppe(n) in an sich bekannter Weise, z.B. mittels Reduktion oder Solvolyse, insbesondere Hydrolyse, bevorzugt in saurem Medium, Alkoholyse, Acidolyse oder Hydrazinolyse. Niederalkoxycarbonyl, wie die tert-Butyloxycarbonylgruppe, oder den Tritylrest kann man beispielsweise durch Behandeln mit einer Säure, wie Mineralsäure, z.B. Halogenwasserstoffsäure, wie Chlorwasserstoffsäure, in An- oder Abwesenheit von Lösungsmitteln, insbesondere Methanol oder Tetrahydrofuran, oder einer organischen Säure, z.B. Ameisen-, Essig- oder Trifluoressigsäure, in An- oder Abwesenheit von Wasser oder eines organischen Lösungsmittels, z.B. Methylenchlorid, freisetzen. Die Abspaltung der gegebenenfalls substituierten Benzyloxycarbonylgruppe gelingt beispielsweise reduktiv durch Hydrogenolyse, d.h. durch Behandeln mit Wasserstoff in Gegenwart eines geeigneten Katalysators, z.B. Palladium, oder mittels Natrium in flüssigem Ammoniak, oder durch Acidolyse, insbesondere mittels Bromwasserstoff/Eisessig. 2-Halogenniederalkoxycarbonyl kann beispielsweise durch Behandeln mit einem geeigneten Reduktionsmittel, wie Zink, in Gegenwart eines organischen Lösungsmittels, wie Methanol oder wässriger Essigsäure, abgespalten werden. Die Abspaltung von Niederalkylformimidoyl, wie tert-Butylformimidoyl, erfolgt vorzugsweise durch Basen, wie Hydroxide, insbesondere Alkalimetallhydroxide, z.B. Kaliumhydroxid. Die Abspaltung der Bisacylreste, insbesondere der Phthalylgruppe, kann beispielsweise mittels Hydrazinhydrat geschehen oder mittels einer Säure, insbesondere einer Mineralsäure, z.B. einer Halogenwasserstoffsäure, wie Chlorwasserstoffsäure, in An- oder Abwesenheit von organischen Lösungsmitteln, z.B. Alkoholen, wie Methanol.

Verfahren (b):

In einer Verbindung der Formel V ist ein Rest $W_1$ (Va) oder $W_2$ (Vb) eine Abgangsgruppe, vorzugsweise eine derivatisierte Hydroxygruppe, z.B. aliphatisch - oder aromatischsubstituiertes Sulfonyloxy, wie Niederalkansulfonyloxy, z.B. Methansulfonyloxy, oder Niederalkylphenylsulfonyloxy (= Niederalkylphenyl-$SO_2$-O-), wie p-Toluolsulfonyloxy, eine derivatisierte Sulfonylgruppe, wie Halogenniederalkansulfonyl, z.B. Trifluormethansulfonyl, oder insbesondere eine freie Hydroxygruppe oder ein Halogenatom, z.B. Chlor, Brom oder Iod.

Bedeutet $W_1$ oder $W_2$ Hydroxy, so erfolgt die Umsetzung vorzugsweise durch eine intramolekulare Dehydratisierungsreaktion. Insbesondere kommt hierfür eine Variante der Mitsunobu-Reaktion (vgl. Synthesis, 682 (1976)) in Frage, bei der die Verbindung der Formel V mit einem aminogeschützten Hydroxylamin-Derivat, worin die Aminofunktion durch eine der unter Verfahren (a) genannten bivalenten Aminoschutzgruppen geschützt ist, z.B. N-Hydroxyphthalimid, N-Hydroxy-5-norbornen-2,3-dicarbonsäureimid oder Acethydroxamsäureethylester, insbesondere N-Hydroxyphthalimid, und Triarylphosphin, z.B. Triphenylphosphin, und einem N,N'-Azodicarbonsäurediester, wie einem N,N'-Azodicarbonsäurediniederalkylester, z.B.

N,N'-Azodicarbonsäurediethylester, umgesetzt wird, vorzugsweise in einem aprotischen Lösungsmittel, wie einem Ether, z.B. einem cyclischen Ether, wie Tetrahydrofuran, oder insbesondere einem aromatischen Lösungsmittel, wie Benzol oder Toluol, vorzugsweise unter Inertgas, wie Stickstoff, und bei bevorzugten Temperaturen von 0 °C bis 80 °C, insbesondere von 10 bis 40 °C, z.B. bei 20 bis 30 °C. Diese Reaktion erfolgt vorzugsweise so, dass am die Hydroxygruppe tragenden Kohlenstoffatom Inversion erfolgt.

Die Reaktion ergibt eine durch eine bivalente Aminoschutzgruppe, insbesondere einen Bisacylrest, geschützte Gruppe der Formel $-O-NH_2$ als $R_1'$ oder $R_2'$ in einer Verbindung der Formel IV, welche wie unter Verfahren (a) freigesetzt werden kann, vorzugsweise durch Hydrolyse, insbesondere in wässriger Lösung in Gegenwart einer Säure, in erster Linie einer Mineralsäure, z. B. einer Halogenwasserstoffsäure, wie Chlorwasserstoffsäure, in An- oder Abwesenheit von organischen Lösungsmitteln, z. B. Alkoholen, wie Methanol, bei Temperaturen zwischen Raumtemperatur und der Rückflusstemperatur des jeweiligen Reaktionsgemisches, insbesondere zwischen 80 °C und der Rückflusstemperatur, z.B. bei Rückflusstemperatur.

Bedeutet $W_1$ oder $W_2$ ein derivatisiertes Hydroxy oder insbesondere ein Halogenatom, z. B. ein Bromatom, so erfolgt die Umsetzung vorzugsweise mit einem aminogeschützten Hydroxylamin-Derivat (wie in Synthetic Communications 19, 339 (1989)), insbesondere einer Hydroxamsäure, wie Benzoylhydroxamsäure, in einem aprotischen Lösungsmittel, vorzugsweise einem Carbonsäureamid, wie einem N,N-Di-niederalkyl-niederalkanoylamid, z. B. Formamid, unter Zugabe eines Alkoholates, wie eines Alkalimetall-niederalkyloxides, z. B. Natriummethoxid, bei Temperaturen zwischen 0 und 80 °C, insbesondere zwischen 30 und 40 °C, z. B. bei Raumtemperatur, in An- oder Abwesenheit eines Inertgases, wie Stickstoff.

Man erhält so eine durch eine Aminoschutzgruppe, insbesondere einen unsubstituierten oder substituierten Benzoylrest, wie unter Verfahren (a) definiert, geschützte Gruppe der Formel $-O-NH_2$ als $R_1'$ oder $R_1'$ in einer Verbindung der Formel IV, welche wie unter Verfahren (a) beschrieben freigesetzt werden kann, vorzugsweise, wie zuletzt für durch eine bivalente Schutzgruppe geschütztes $-O-NH_2$ beschrieben.

Weitere funktionelle Gruppen in Ausgangsmaterialien, die nicht an der Umwandlung teilnehmen sollen, sind in erster Linie primäre und sekundäre Aminogruppen und können durch geeignete Schutzgruppen (conventional protecting groups) geschützt werden, insbesondere durch Aminoschutzgruppen (wie in einer durch eine Aminoschutzgruppe als $X_1$ am Piperidin-Stickstoff geschützten Verbindung der Formel V), wie oben unter Verfahren (a) beschrieben.

Die Abspaltung von Schutzgruppen, insbesondere die Freisetzung der geschützten Aminogruppen nach der Umwandlung von $W_1$ oder $W_2$, erfolgt vorzugsweise wie unter Verfahren (a) beschrieben.

Zusätzliche Verfahrensmassnahmen

Die Überführung einer Verbindung der Formel I in eine andere kann beispielsweise dadurch erfolgen, dass in einer Verbindung der Formel I, in der nur ein Rest R vorliegt, ein weiterer Rest R eingeführt wird. So kann eine Verbindung der Fomel II, worin die Reste die genannten Bedeutungen haben, in eine Verbindung der Formel III überführt werden, indem man zunächst Aminoschutzgruppen einführt, wie unter Verfahren (a) beschrieben, wobei am Piperidinstickstoff ein Acylrest eines Kohlensäurehalbesters, wie tert-Niederalkoxycarbonyl, insbesondere tert-Butoxycarbonyl, oder ein Niederalkylformimidoylrest, wie tert-Butyl-formimidoyl, als Schutzgruppe besonders bevorzugt ist. Anschliessend erfolgt Umsetzung mit einer Niederalkyl-Lithiumverbindung, wie tert-Butyllithium, die in einem cyclischen, linearen oder verzweigten Kohlenwasserstoff oder einem Gemisch von Kohlenwasserstoffen dieser Art, z.B. Cyclohexan:Isopentan, gelöst ist, in Gegenwart einer tertiären Stickstoffbase, wie N,N,N',N'-Tetramethylethylendiamin in einem aprotischen Lösungsmittel, z.B. Ether, wie Dimethylether, Tetrahydrofuran oder einem Gemisch davon, bei Temperaturen zwischen -100 und -20 °C, insbesondere von etwa -60 bis etwa -75 °C, wobei ein Derivat der geschützten Verbindung der Formel I erhalten wird, welches an dem Ringkohlenstoffatom lithiiert ist, das dem Ringstickstoff des Piperidinringsystems benachbart ist. Dieses Derivat wird dann vorzugsweise gleich in situ weiterverarbeitet, indem man es mit einem stark elektrophilen Methyl- oder Ethylderivat umsetzt, z. B. Methyl- oder Ethyljodid, Dimethylsulfat oder Diethylsulfat, in einem aprotischen Lösungsmittel, wie gerade definiert, bei den zuletzt genannten Temperaturen. Die Verbindung der Formel III wird dann durch Freisetzung der Aminogruppen erhalten, wie oben unter Verfahren (a) beschrieben.

Isomerengemische von Verbindungen der Formel I, die in Form mehrerer Isomeren vorliegen können, können nach an sich bekannten Verfahren in die einzelnen Isomeren oder in Isomerengemische aufgetrennt werden, z.B. in Diastereomere (z. B. bezüglich der cis- oder trans-Stellung von Substituenten), Racemate oder Enantiomere.

Gemische von Diastereomeren können beispielsweise in einzelne Diastereomeren aufgetrennt werden, vorzugsweise chromatographisch, z. B. durch Verteilungs- oder Adsorptionschromatographie, oder durch Verteilung in mehrphasigen Lösungsmittelgemischen.

Gemische von Enantiomeren können beipielsweise in einzelne Enantiomere aufgetrennt werden, vorzugsweise durch Bilden von Salzen mit optisch reinen salzbildenden Reagentien und Auftrennen des so erhältlichen Diastereomerengemisches, z.B. mittels fraktionierter Kristallisation, oder durch Chromatographie an optisch aktiven Säulenmaterialien.

Ferner können auch aus Verbindungen der Formel I Verbindungen der Formel IV zu Reinigungszwecken hergestellt werden. Die Freisetzung der gereinigten Verbindungen der Formel I erfolgt dann durch Schutzgruppenabspaltung, wie unter Verfahren (a) beschrieben.

Je nach Verfahrensweise bzw. Reaktionsbedingungen können die erfindungsgemässen Verbindungen mit salzbildenden basischen Gruppen in freier Form oder in Form von Salzen erhalten werden.

Die Verbindungen, einschliesslich ihrer Salze, können auch in Form ihrer Hydrate erhalten werden, oder ihre Kristalle können z.B. das zur Kristallisation verwendete Lösungsmittel einschliessen.

Salze von freien Verbindungen der Formel (I) können in an sich bekannter Weise hergestellt werden, beispielsweise durch Behandeln mit einer Säure, wie einer anorganischen Säure, z. B. Salzsäure oder Schwefelsäure, einer organischen Carbonsäure, z. B. Adipinsäure, oder einer organischen Sulfonsäure, z. B. Benzolsulfonsäure oder einem geeigneten Anionenaustauscherreagenz, welches beispielsweise mit dem Anion der entsprechenden Säure beladen ist. Salze können in üblicher Weise in die freien Verbindungen überführt werden, z.B. durch Behandeln mit einem geeigneten basischen Mittel, wie einer Hydroxybase in freier Lösung, z. B. einem Alkalimetallhydroxid, oder wie einem mit Hydroxid beladenen Anionenaustauscher, z. B. durch Chromatographie oder im Batch-Verfahren.

Die Umwandlung eines Salzes einer Verbindung der Formel I kann über die Gewinnung der freien Verbindung und deren anschliessende Umsetzung zu einem Säureadditionssalz erfolgen, wie gerade beschrieben.

Auch ist die direkte Umwandlung eines Säureadditionssalzes aus einer der Verbindungen der Formel I und einer Säure mit einer anderen Säure in ein Säureadditionssalz aus der Verbindung der Formel I und der zweiten, neuen Säure möglich. Diese Umwandlung erfolgt vorzugsweise a) durch Reaktion des ursprünglichen Säureadditionssalzes in freier Lösung in Gegenwart einer geeigneten Menge der neuen Säure, z. B. einem Überschuss, oder b) an einem mit dem Anion der neuen Säure beladenen Anionenaustauscher.

Für alle Reaktionen, die der Umwandlung von Säureadditionssalzen von Basen der Formel I in andere Säureadditionssalze oder in die freien Verbindungen oder von den freien Basen in die entsprechenden Säuren dienen, sind auch gelchromatographische Umsalzungsverfahren anwendbar.

Die Umwandlung eines Salzes, vorzugsweise eines Halogenides, wie Chlorides, in ein anderes, beispielsweise ein Salz einer zweifach negativ geladenen Säure, z. B. ein Sulfat, ist insbesondere dann zu bevorzugen, wenn so ein kristallines Salz einer Verbindung der Formel I erhalten wird.

Ausgangsmaterialien

Die Ausgangsverbindungen der Formel V sind bekannt oder werden nach an sich bekannten Verfahren hergestellt.

Beispielsweise können sie

(A) durch Hydrierung entsprechend substituierter Pyridine der Formel VI,

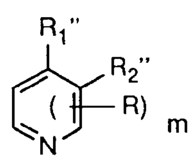

(VI)

worin die Reste die für Verbindungen der Formel V genannten Bedeutungen haben, vorzugsweise mit Wasserstoff in Gegenwart von Katalysatoren, insbesondere Pt-Pd-Mischbettkatalysatoren in Carbonsäureanhydriden, wie Acetanhydrid, Rhodium/Kohle-Katalysatoren in Alkoholen, wie Ethanol, Platin-, Rhodium-, Palladium-, Nickel-, Ruthenium-Katalysatoren, Nickel auf Silikatträgem, Raney-Nickel oder Palladium/Kohle-Katalysatoren, unter atmosphärischem oder erhöhtem Druck, hergestellt werden - Beispiele für eine Verbindung der Formel VI sind 3,5-Dimethyl- oder 3,5-Diethyl-4-hydroxy-pyridin, welches durch Erhitzen von entsprechend substituierten Bipyridyliumsalzen mit Phosphorsäure gewonnen wird (Synthesis, 454 (1979)), 3-Methyl-4-hydroxy-pyridin, welches beim Erwärmen von 3-Methyl-4-pyridylamin in wässriger Schwefelsäure und wässrigem Natriumnitrit über das Diazoniumsalz (Archiv der Pharmazie 290, 494, 509 (1957)) oder beim Erhitzen von 4-Hydroxy-5-methyl-nicotinsäure unter Decarboxylierung (ebendort, S. 508) erhalten wird, und 2,3-Dimethyl-4-hydroxypyridin, welches aus 2,3-Dimethyl-4-nitropyridin mit Kaliumacetat in Acetanhydrid bei Rückflusstemperatur und anschliessende Hydrolyse des erhaltenen 4-Acetoxy-2,3-dimethoxypyridins gewonnen wird (J. Org. Chem. 44, 870 (1979)-, oder sie können,
(B) soweit es sich um Verbindungen der Formel V handelt, die in 4-Stellung als $R_1$" eine Hydroxygruppe enthalten und als $R_2$" Wasserstoff, durch Herstellung aus entsprechend substituierten γ-Pyronen der Formel VII,

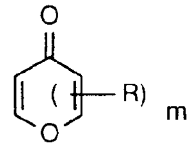

(VII)

worin R und m die für Verbindungen der Formel I genannten Bedeutungen haben, gewonnen werden, wobei diese zunächst in Gegenwart von Ammoniak in Alkoholen, wie Ethanol (J. Chem. Soc., 3023 (1931)), in die entsprechenden γ-Pyridone umgewandelt werden und anschliessend zu den entsprechenden 4-Hydroxypiperidinen der Formel V hydriert werden, beispielsweise, wie unter (A) für die Pyridine beschrieben, oder direkt durch Reduktion in Gegenwart von Ammoniak, beispielsweise mit Natrium oder durch Hydrierung in Gegenwart von Platin oder Palladium in 4-Hydroxypiperidine umgewandelt werden - Beispiele für Verbindungen der Formel VII sind 2,6-Dimethyl-γ-pyron, das aus Dehydroessigsäure hergestellt werden kann (Bull. Chem. Soc. Japan 48, 508 (1975), 2,6-Diethyl-γ-pyron, das beim Erhitzen von 6-Ethyl-3-propionyl-pyran-2,4-dion mit wässriger Salzsäure entsteht (J. Indian Chem. Soc. 9, 303, 305 (1932)), 2-Methyl-3-ethyl-γ-pyron und 2,3-Dimethyl-γ-pyron, welche beispielsweise hergestellt werden durch Erhitzen von 3-Ethyl-bzw. 3-Methyl-2,4-pentandion in Ethylenglykol und mit p-Toluolsulfonsäure als Katalysator auf Rückflusstemperatur über das entsprechende Ketal (3-Ethyl-bzw. 3-Methyl-4,4-(ethylendioxy)pentan-2-on), welches dann etwa bei Raumtemperatur acetyliert wird in Gegenwart von Diethyloxalat und Natriummethoxid in wasserfreiem Methanol zu Ethyl-2,4-diketo-5-ethyl- oder -methyl-6,6-(ethylendioxy-)heptanoat, das in wässriger Chlorwasserstoff-Lösung hydrolysiert wird zu 2-Methyl-3-ethyl- oder 2,3-Dimethyl-4-pyron-6-carbonsäure, welche dann unter Erhitzen auf 240 °C mit Kupferpulver unter Atmosphärendruck decarboxyliert werden zu den genannten Pyronen (J. Org. Chem. 49, 4523 (1984), oder 3-Methyl-γ-pyron, welches beispielsweise aus dem Kaliumsalz des Monomethylethers von Bis-oxymethylen-aceton in methanolischer Lösung und Methyliodid gewonnen werden kann (Willstätter und Pummerer, Chemische Berichte 38, 1461 (1905) -, oder sie können
(C) durch Cyclisierung von Verbindungen der Formel VIII,

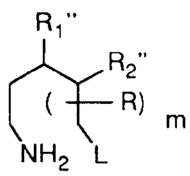

(VIII)

worin L eine nukleofuge Abgangsgruppe bedeutet, beispielsweise eine der oben bei der Definition von $W_1$ und $W_2$ unter Verfahren (b) genannten, insbesondere eine Aminogruppe, und die übrigen Reste die für Verbindungen der Formel V genannten Bedeutungen haben, erhalten werden, wobei beispielsweise beim Vorliegen einer Aminogruppe L diese und die weitere Aminogruppe vorzugsweise in protonierter Form vorliegen, z. B. als Salz einer Halogenwasserstoffsäure, wie Chlorwasserstoffsäure, und die Umsetzung durch Erhitzen bewirkt wird, oder sie können,
(D) sofern es sich um Verbindungen der Formel V handelt, die in 4-Stellung als Rest $R_1$" eine Hydroxygruppe und als $R_2$" Wasserstoff enthalten, durch Cyclisierung von Verbindungen der Formel IX,

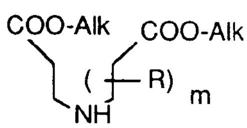

(IX)

worin Alk einen Niederalkylrest, insbesondere Methyl oder Ethyl, bedeutet und die übrigen Reste die für Verbindungen der Formel I genannten Bedeutungen haben, hergestellt werden unter Basenkatalyse analog der Dieckmann-Methode mit anschliessender Hydrolyse und Decarboxylierung zu den entsprechenden 4-Piperidonen (vgl. u. a. J. Am. Chem. Soc. 51, 924 (1929); J. Am. Chem. Soc. 70, 1820, 1826 (1948), die dann hydriert werden zu den 4-Hydroxypiperidinen der Formel V, beispielsweise unter den bei (A) beschriebenen Hydrierungsbedingungen, oder mit komplexen Hydriden, beispielsweise Natriumborhydrid in alkoholischer Lösung, wie Methanol, oder mit Natrium in absoluten Alkoholen, wie Ethanol, oder sie können,

(E) sofern es sich um Verbindungen der Formel V handelt, in denen $R_1$" Hydroxy und $R_2$" Wasserstoff bedeutet, m gleich 2 ist und die beiden Reste R an die beiden dem Stickstoff benachbarten Kohlenstoffatome gebunden sind, wobei beide Reste R identisch sind, durch Cyclisierung von 2 Mol Acetaldehyd oder Propionaldehyd, einem Mol eines Acetondicarbonsäureesters und Ammoniak oder einem primären Amin analog der Methode von Petrenko-Kritschenko synthetisiert werden, wobei 4-Piperidone erhalten werden, die dann unter Hydrierungsbedingungen wie unter (A) oder mit komplexen Hydriden, beispielsweise Natriumborhydrid in alkoholischer Lösung, wie Methanol, oder mit Natrium in absoluten Alkoholen, wie Ethanol, zu den entsprechenden 4-Hydroxyverbindungen umgewandelt werden können, oder sie können

(F) durch ein Verfahren analog der Hantzschen Pyridinsynthese gewonnen werden durch Kondensation von 2 Mol einer entsprechend substituierten β-Dicarbonylverbindung mit einem Mol eines Aldehyds in Gegenwart von Ammoniak, wobei die entsprechenden Dihydropyridinderivate erhalten werden, anschliessende Oxidation dieser Verbindungen zu den entsprechenden Pyridinderivaten und Decarboxylierung, beispielsweise durch Hydrolyse der Ester und Erhitzen, und schliesslich Hydrierung der so erhaltenen substituierten Pyridine, wie unter (A) beschrieben, oder sie können,

(G) sofern es sich um Verbindungen der Formel V handelt, worin R nicht an ein dem Ringstickstoff des Piperidinringsystems benachbartes Kohlenstoffatom gebunden ist, durch Reaktion von 1,5-Dicarbonylverbindungen der Formel X,

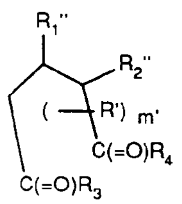

(X)

worin $R_1$" und $R_2$" die für Verbindungen der Formel I genannten Bedeutungen haben, $R_3$ und $R_4$ unabhängig voneinander Wasserstoff, Methyl oder Ethyl bedeuten, R' Methyl oder Ethyl bedeutet und nicht direkt an eines der Carbonyl-Kohlenstoffatome gebunden ist, und m' 0, 1 oder 2 bedeutet mit der Massgabe, dass m' 0 ist, wenn beide Reste $R_3$ und $R_4$ eine andere Bedeutung als Wasserstoff haben, oder m' 0 oder 1 ist, wenn nur einer der Reste $R_3$ oder $R_4$ eine andere Bedeutung als Wasserstoff hat, oder m' 1 oder 2 ist, wenn beide Reste $R_3$ und $R_4$ Wasserstoff bedeuten, erhalten werden, beispielsweise, wenn man diese Verbindungen einem hydrierenden Ringschluss in Gegenwart von Ammoniak unterwirft (1,5-Dialdehyde sind hierbei bevorzugte Ausgangsmaterialien ($R_3$=$R_4$= H)) - bei-spielsweise kann man 3-(2-Hydroxyethyl)piperidin durch Hydrierung in Gegenwart von Raney-Nickel, Wasserstoff und Ammoniak aus 2-Hydroxyethylglutardialdehyd herstellen (Bulletin de la Société Chimique de France, Seite 1139 (1954)), in das dann Methyl- oder Ethylreste in 2- oder 6-Stellung eingeführt werden können, wie bei der Umwandlung von Verbindungen der Formel II in solche der Formel III beschrieben, oder sie können,

(H) sofern es sich um Verbindungen der Formel V handelt, worin im Falle der Definition des Restes $R_1$ durch das Radikal der Formel Ia n 1 ist, oder im Falle der Definition von $R_2$ durch das Radikal der Formel Vb p 1 oder 2 ist, durch Reduktion von Estern der Formel XI,

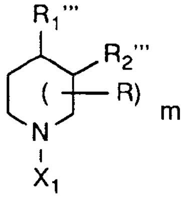

(XI)

worin entweder $R_1$''' eine veresterte Carboxygruppe, insbesondere eine Alkyl-, Aryl- oder Arylniederalkyloxycarbonylgruppe, z. B. Niederalkoxycarbonyl, wie Methoxycarbonyl, Ethoxycarbonyl oder n-Butoxycarbonyl, 4-Nitrophenyloxycarbonyl oder Benzyloxycarbonyl und $R_2'''$ ein Wasserstoffatom bedeuten, oder $R_1'''$ ein Wasserstoffatom und $R_2'''$ eine veresterte Carboxygruppe oder eine veresterte Carboxymethylgruppe bedeuten, insbesondere eine Alkyl-, Aryl- oder Arylniederalkyl-oxycarbonylgruppe oder eine Alkyl-, Aryl- oder Arylniederalkyl-oxycarbonylmethylgruppe, z. B. Niederalkoxycarbonyl, wie Methoxy-, Ethoxy- oder n-Butoxycarbonyl, oder Niederalkoxycarbonylmethyl, wie Methoxy-, Ethoxy- oder n-Butoxycarbonylmethyl, und die übrigen Reste die für Verbindungen der Formel V genannten Bedeutungen haben, mit komplexen Hydriden, insbesondere Lithiumaluminiumhydrid in Alkoholen, wie Ethanol, oder Natriumborhydrid in Gegenwart von LiCl in Diglycol oder $LiAlH[OC(CH_3)_3]_3$ in Ethern, wie Tetrahydrofuran, gewonnen werden - Beispiele sind 2-(2-Methyl-[3]piperidyl)ethanol, das aus 7a-Methyl-2,3,3a,7a-tetrahydro-4*H*-furo[2,3-b]pyran (hergestellt aus Acrylaldehyd und 5-Methyl-2,3-dihydrofuran) durch Umsetzen mit wässriger Chlorwasserstofflösung, Sättigen der Reaktionslösung mit Ammoniak und anschliessendes Hydrieren mit Wasserstoff an Raney-Nickel bei 100 °C/100 bar in Gegenwart geringer Mengen Natronlauge (Bulletin de la Société Chimique de France, Seite 1139, 1142 (1954)), alternativ durch Erhitzen von (2-Methyl-6-oxo-[4]piperidyl)-essigsäure-ethylester mit Lithiumalanat in Ether (Collections of Czechoslowak Chemical Communications 29, Seite 1582, 1587 (1964)), oder durch Erhitzen von (4-Methyl-2,6-dioxo-[4]piperidyl)-essigsäure-butylester mit Lithiumalanat in Dibutylether (Collections of Czechoslowak Chemical Communications 31, 4592 (1966)), hergestellt werden kann; 2-Methyl-[3]piperidylmethanol, welches aus 2-Methyl-[3]piperidylcarbonsäureethylester (hergestellt aus 3-Amino-2-[2-cyanoethyl]-crotonsäure-ethylester durch Hydrierung an Nickel/Fullererde in Ethanol (Chemische Berichte 82, Seite 104 (1949)) oder aus 2-[2-Cyanoethyl]acetessigsäure-ethylester an Raney-Nickel in Ethanol (J. Amer. Chem. Soc. 72, Seite 2594, 2596 (1950)) durch Reduktion mit komplexen Hydriden erhalten werden kann; 4-Methyl- oder 5-Methyl-[3]piperidylmethanol, welche durch Hydrierung von 2-Cyano-3-methyl-glutarsäurediethylester oder 2-Cyano-4-methyl-glutarsäurediethylester in ethanolischem Chlorwasserstoff in Gegenwart von Platindioxid, Reduktion des erhaltenen 4- oder 5-Methyl-5-carbethoxy-2-piperidons nach der Methode von Borch mit NaBH4 in Gegenwart von $Me_3OBF_4$ in Methylenchlorid bei Temperaturen unter 10 °C zum 4- bzw. 5-Methyl-[3]piperidylcarbonsäureethylester (Bull. Soc. Chim. France, Seite 663 (1986) (4)) und deren Reduktion mit komplexen Hydriden erhalten werden; oder 6-Methyl-[3]piperidylmethanol, welches durch Hydrolyse von 6-Methyl-3-cyanopyridin mit Schwefelsäure in Ethanol bei Rückflusstemperatur, Hydrierung des erhaltenen 6-Methyl-[3]pyridincarbonsäureethylesters in mit Chlorwasserstoff gesättigtem Ethanol in Gegenwart von Platindioxid (Bull. Soc. Chim. France, Seite 663 (1986) (4)) und Reduktion des erhaltenen 6-Methyl-[3]piperidylcarbonsäureethylesters mit komplexen Hydriden erhalten wird.

Neben diesen allgemeineren Herstellungsmethoden gibt es eine Reihe weiterer Verfahren zur Herstellung spezieller Verbindungen der Formel I, von denen im folgenden einige genannt sein sollen, die sich in geeigneter Weise verallgemeinern lassen.

Beispielsweise kann man 3-Methyl-piperidin-4-ol der Formel V aus N-Benzyl-3-methyl-piperidin-4-ol durch Schütteln mit Palladium/Holzkohle in Ethanol mit Wasserstoff herstellen (Can. J. Chem. 50, 803 (1972), oder durch Reduktion von 3-Methyl-piperidin-4-on mit Natriumborhydrid in Gegenwart von Kaliumhydroxid in wässriger Lösung (Bull. Acad. Sci. USSR, Div. Chem. Sci. (englische Version), Seite 1788 (1965)); 2,5-Dimethyl-piperidin-4-ol der Formel V durch Reduktion von 2,5-Dimethyl-piperidin-4-on mit Natrium in Ethanol oder Wasserstoff in Gegenwart eines Platinkatalysators (Bull. Acad. Sci. USSR, Div. Chem. Sci. (englische Version), Seite 65 (1954)); 4-Methyl-3-hydroxymethyl-piperidin der Formel V durch Aminomerkurierung von 2-Aminomethyl-3-methyl-4-penten-1-ol mit Quecksilberacetat in Tetrahydrofuran/Wasser mit anschliessender reduktiver Entfernung des gebundenen Quecksilberradikals mit Natriumborhydrid (J. Het. Chem. 9, Seite 1081 (1972)); und 2,6-Diethyl- oder 2,6-Dimethyl-3-hydroxymethyl-piperidin aus N-[(1-Ethyl- oder 1-Methyl-)-4-pentenyl]nitronen über das entsprechende 2-endo,8-exo-Diethyl- oder 2-endo,8-exo-Dimethyl-7-oxa-1-azabicyclo-[3.2.1]octan mit Zinkstaub in wässriger Essigsäure, wobei die all-cis-Form erhalten wird (J. Am. Chem. Soc. 111, 3363 (1989)).

Besonders bevorzugt zur Herstellung von solchen Verbindungen der Formel V, worin $R_1''$ eine Hydroxygruppe und $R_2''$ ein Wasserstoffatom bedeutet, $X_1$ eine Aminoschutzgruppe bedeutet und die übrigen Reste die genannten Bedeutungen haben, wobei wenigstens einer der Reste R am dem Piperidin-Ringstickstoff benachbarten Ringkohlentoffatom gebunden ist, ist das folgende Verfahren:

Zunächst wird eine Verbindung der Formel XII,

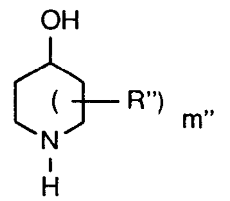

(XII)

worin R" $C_1$-$C_2$-Alkyl bedeutet und m" 0 oder 1 ist, durch eine unter Verfahren (a) beschriebene Aminoschutzgruppe geschützt, beispielsweise durch einen Acylrest eines Kohlensäurehalbesters, vorzugsweise in 1-Stellung des Niederalkylrestes verzweigtes Niederalkoxycarbonyl, wie tert-Butoxycarbonyl, indem man die Verbindung der Formel XII mit einem aktivierten Derivat des Kohlensäurehalbesters, vorzugsweise einem Anhydrid oder einem Säurehalogenid, wie Di-tert-butyl-dicarbonat oder tert-Butyloxycarbonylchlorid, in einem aprotischen Lösungsmittel, wie einem halogenierten Kohlenwasserstoff, z. B. Methylenchlorid, bei Temperaturen von 20 °C bis zur Rückflusstemperatur des Reaktionsgemisches umsetzt, und, vorzugsweise anschliessend, das erhaltene N-acylierte Produkt unter Einführung einer Hydroxyschutzgruppe, wie weiter unten beschrieben, insbesondere einer unter anderen Bedingungen als die Aminoschutzgruppe abspaltbaren Hydroxyschutzgruppe, vorzugsweise 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, umsetzt, beispielsweise nach einer der in den unten genannten Standardwerken aufgeführten Methoden, vorzugsweise im Falle von 2-Oxa- oder 2-Thiacycloalkyl durch Umsetzung einer entsprechenden einfach 2-ungesättigten 2-Oxa oder 2-Thiacycloalkylverbindung, insbesondere 3,4-Dihydro-2H-pyran, in einem aprotischen Lösungsmittel, wie einem halogenierten Kohlenwasserstoff z. B. Methylenchlorid, in Gegenwart einer schwach sauren Verbindung, z. B. eines Pyridiniumsalzes, wie Pyridinium-(toluol-4-sulfonat), bei Temperaturen von 0 bis 50 °C, insbesondere bei Raumtemperatur, die erhaltene Verbindung der Formel XIII,

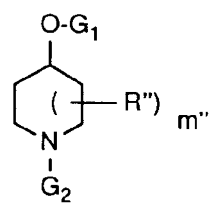

(XIII)

worin $G_1$ eine Hydroxyschutzgruppe und $G_2$ eine Aminoschutzgruppe bedeutet, wie zuletzt beschrieben, und die übrigen Symbole die für Verbindungen der Formel XII angegebenen Bedeutungen haben, mit einer Lithiumalkylverbindung, z. B. sec-Niederalkyllithium, wie sec-Butyllithium, welche vorzugsweise gelöst in einem cyclischen, einem linearen oder einem verzweigten Kohlenwasserstoff oder einem Gemisch von Kohlenwasserstoffen dieser Art, z. B. Cyclohexan:Isopentan, zugesetzt wird, in Gegenwart einer tertiären Stickstoffbase, wie eines Mono- oder Di-(N,N-diniederalkyl-amino)niederalkans, z.B. N,N,N',N'-Tetramethylethylenediamin, vorzugsweise unter Inertgas, wie Argon oder Stickstoff, in einem aprotischen Lösungsmittel, besonders bevorzugt einem Ether, wie Diethylether, bei Temperaturen zwischen 0 und -100 °C, vorzugsweise zwischen -60 und -80 °C, umsetzt. Die erhaltene lithiierte Verbindung wird vorzugsweise direkt in situ im selben Lösungsmittel und bei gleichen Bedingungen, wie für die Lithiierung beschrieben, unter Einführung von einem oder, insbesondere nach Isolierung des monoalkylierten Zwischenproduktes und Herstellung von dessen lithiiertem Derivat analog der gerade beschriebenen Methode, zwei $C_1$-$C_2$-Alkylresten umgesetzt. Hierfür setzt man eine Verbindung der Formel XIV,

($C_1$-$C_2$-Alkyl)-Y (XIV),

worin Y eine nukleofuge Abgangsgruppe, beispielsweise, wie unter Verfahren (b) beschrieben, z. B. aliphatisch - oder aromatisch - substituiertes Sulfonyloxy, wie Niederalkansulfonyloxy, z. B. Methansulfonyloxy, oder Niederalkylphenylsulfonyloxy (= Niederalkylphenyl-$SO_2$-O-), wie p-Toluolsulfonyloxy, oder insbesondere ein Halogenatom, z.B. Chlor, Brom oder vorzugsweise Iod, bedeutet und $C_1$-$C_2$-Alkyl Methyl oder Ethyl bedeutet, oder ein Di-($C_1$-$C_2$-alkyl)sulfat, insbesondere Dimethylsulfat oder Diethylsulfat, oder Fluorsulfonsäuremethylester ein, wobei eine Verbindung der Formel XV

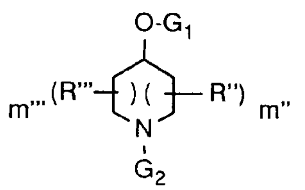

(XV)

erhalten wird, worin R''' $C_1$-$C_2$-Alkyl bedeutet und in 2-, 6- oder 2- und 6-Stellung des zentralen Piperidinringes gebunden ist, m''' 1 oder 2 ist und die übrigen Reste die genannten Bedeutungen haben, mit der Massgabe, dass die Addition von m'' und m''' 1 oder 2 ergibt, dann aus der Verbindung der Fomel XV die Hydroxyschutzgruppe $G_1$ abspaltet, vorzugsweise selektiv, im Falle der 2-Oxa- oder 2-Thiacycloalkyl-Schutzgruppen mit 5-7 Ringatomen, wie 2-Tetrahydrofuryl oder 2-Tetrahydropyranyl oder eines entsprechenden Thiaanalogen z. B. unter mild sauren Bedingungen, wie in Gegenwart eines Kationenaustauschers in der $H^+$-Form, vorzugsweise in Alkoholen, wie Methanol oder Ethanol, bei Temperaturen zwischen 0 und 60 °C, z. B. bei Raumtemperatur. Man erhält eine Verbindung der Formel XVI,

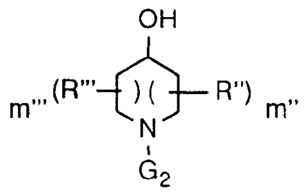

(XVI),

worin die Reste die zuletzt genannten Bedeutungen haben und die einer Verbindung der Formel V entspricht, worin $R_1$'' eine Hydroxygruppe und $R_2$'' ein Wasserstoffatom bedeuten, $X_1$ eine Aminoschutzgruppe bedeutet und die übrigen Reste die genannten Bedeutungen haben, wobei wenigstens einer der Reste R am dem Piperidin-Ringstickstoff benachbarten Ringkohlentoffatom gebunden ist.

Gewünschtenfalls wird, sofern ein Isomeres oder ein Isomerengemisch der Verbindung der Formel XVI erhalten wird, bei welchem die Konfiguration an dem Kohlenstoffatom, welches die Hydroxygruppe trägt, einer Inversion unterzogen werden soll, durch eine intramolekulare Dehydratisierungsreaktion, insbesondere eine Variante der Mitsunobu-Reaktion (vgl. Tetrahedron Lett. 32, 3017 (1991)), bei der die Verbindung der Formel XVI zunächst mit einer aciden Carboxyverbindung, insbesondere einer durch elektronenziehende Substituenten aktivierten Arylcarbonsäure, wie durch 1 bis 3 Nitro-, Fluor-, Chlor- oder Brom-radikale vorzugsweise m- oder p-substituierte Benzoesäure, oder einer 2-halogenierten Niederalkancarbonsäure, z. B. 2,2,2-Trichlor- oder 2,2,2-Trifluoressigsäure, vor allem 4-Nitrobenzoesäure, in Gegenwart von einem Triarylphosphin, z.B. Triphenylphosphin, und einem N,N'-Azodicarbonsäurediester, wie einem N,N'-Azodicarbonsäurediniederalkylester, z. B. N,N'-Azodicarbonsäurediethylester, umgesetzt, vorzugsweise in einem aprotischen Lösungsmittel, wie einem Ether, z. B. einem cyclischen Ether, wie Tetrahydrofuran, oder insbesondere einem aromatischen Lösungsmittel, wie Benzol oder Toluol, vorzugsweise unter Inertgas, wie Stickstoff, und bei bevorzugten Temperaturen von 0 °C bis 80 °C, insbesondere von 10 bis 40 °C, z. B. bei 20 bis 30 °C, ein entsprechendes 4-Acyloxyderivat der Verbindung der Formel XVI hergestellt. Diese Reaktion erfolgt vorzugsweise so, dass am die Hydroxygruppe tragenden Kohlenstoffatom die entsprechende acylierte Hydroxygruppe unter Inversion eingeführt wird. Hieraus wird dann, indem die Acyloxygruppe in eine Hydroxygruppe umgewandelt wird, die ensprechende Verbindung der Formel XVI hergestellt, bei der die Konfiguration am Kohlenstoffatom in 4-Stellung umgekehrt ist. Dies geschieht vorzugsweise selektiv ohne Abspaltung der Aminoschutzgruppe $G_2$, beispielsweise durch Basenkatalyse, z. B. durch Alkalihydroxide, wie Kaliumhydroxid, oder insbesondere durch Umesterung des Acylrestes in Alkoholen, wie Methanol oder Ethanol, in Gegenwart von katalytischen Mengen von Alkali-Alkoholaten, wie Natrium- oder Kalium-methoxylat oder Natrium- oder Kalium-ethoxylat, bei Temperaturen zwischen 0 und 60 °C, z. B. etwa bei Raumtemperatur.

Verbindungen der Formel V, worin $R_1$'' eine Hydroxygruppe und $R_2$'' ein Wasserstoffatom bedeutet, $X_1$ eine Aminoschutzgruppe bedeutet und die übrigen Reste die genannten Bedeutungen haben, wobei wenigstens einer der Reste R an das Ringkohlentoffatom gebunden ist, welches dem Piperidin-Ringstickstoffatom benachbart ist, können auch durch Einführung einer Aminoschutzgruppe in die entsprechenden Verbindungen gewonnen werden, in denen $X_1$ Wasserstoff bedeutet, beispielsweise in analoger Weise, wie oben bei der Einführung der Aminoschutzgruppe $G_2$ in Verbindungen der Formel XII beschrieben. Hierbei werden ebenfalls Verbindungen der Formel XVI erhalten, wie oben definiert.

Verbindungen der Formel IV mit den oben definierten Resten können allgemein aus Verbindungen der Formel V gewonnen werden, indem der Rest $R_1$" oder $R_2$" in einer Verbindung der Formel V in den Rest $R_1$' oder $R_2$' überführt wird, wie unter Verfahren (b) beschrieben.

In Fortsetzung der als besonders bevorzugt beschriebenen Herstellungsmethode für Verbindungen der Formel V können erhaltene Verbindungen der Formel XVI analog umgesetzt werden, wie es für Verbindungen der Formel V mit einer freien Hydroxygruppe $W_1$ unter Verfahren (b) beschrieben ist. Hierbei entstehen die unter Formel IV fallenden Verbindungen der Formel XVII,

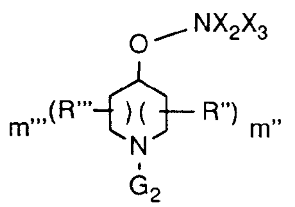

(XVII)

worin $X_2$ und $X_3$ die für Verbindungen der Formel IV in dem Radikal $R_1$' der Formel IVa angegebenen Bedeutungen haben, insbesondere gemeinsam einen Bisacylrest bedeuten, vorzugsweise unsubstituiertes oder, beispielsweise durch dieselben Substituenten, wie oben bei der Definition der Schutzgruppen unter Verfahren (a) für substituiertes Benzoyl definiert, substituiertes Phthalyl, z.B. den Phthalylrest, welcher zusammen mit dem zu schützenden Stickstoffatom ein 1H-Isoindol-1,3(2H)-dion (Phthalimidogruppe) bildet, oder Niederalkyl-dicarbonsäurereste, wie der Bernsteinsäurerest, Niederalkenyldicarbonsäurereste, wie der Maleinsäurerest, oder $C_6$-$C_{12}$-Bicyclodicarbonsäurereste, wie der 5-Norbornen-2,3-dicarbonsäurerest, und die übrigen Reste die für Verbindungen der Formel XVI genannten Bedeutungen haben. Durch Schutzgruppenabspaltung, insbesondere unter sauren Bedingungen, vorzugsweise mit Mineralsäuren, wie Halogenwasserstoffsäuren, in An- oder Abwesenheit von organischen Lösungsmitteln, z.B. Alkoholen, wie Methanol, erhält man aus den Verbindungen der Formel XVII Verbindungen der Formel I, in denen $R_1$ den Rest -O-$NH_2$ bedeutet, $R_2$ ein Wasserstoffatom bedeutet, R Methyl oder Ethyl bedeutet, wenigstens einer der Reste R an das Ringkohlenstoffatom gebunden ist, das dem Piperidin-Ringstockstoff benachbart ist, und m 1 oder 2 ist, oder Salze hiervon.

<u>Generelle Anmerkungen zu den Verfahren:</u>

Alle oben angeführten Verfahrensschritte können unter an sich bekannten, vorzugsweise den spezifisch genannten, Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, z. B. Ionenaustauschern, wie Kationenaustauschern, z. B. in der $H^+$-Form, je nach Art der Reaktion und/oder der Reaktionsteilnehmer bei erniedrigter, normaler oder erhöhter Temperatur, z.B. im Temperaturbereich von etwa -100°C bis etwa 190°C, vorzugsweise von etwa -80 °C bis etwa 150 °C, z.B. bei - 80 bis -60 °C, bei Raumtempemtur, bei - 20 bis 40 °C oder beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Argon- oder Stickstoffatmosphäre.

Bei allen Ausgangs- und Zwischenverbindungen können, sofern salzbildende Gruppen vorliegen, Salze vorliegen. Salze können auch während der Umsetzung derartiger Verbindungen vorliegen, sofern dadurch die Reaktion nicht gestört wird.

Auf allen Reaktionsstufen können auftretende Isomerengemische in die einzelnen Isomeren, z. B. Diastereomeren oder Enantiomeren, oder in beliebige Gemische von Isomeren, z.B. Racemate oder Diastereomerengemische, aufgetrennt werden, beispielsweise analog zu den Methoden, die unter den "Zusätzlichen Verfahrensmassnahmen" beschrieben sind.

In bestimmten Fällen, beispielsweise bei Hydrierungen, bei Mitsunobu-Reaktionen mit Invertierungen, oder bei Lithiierungen, wie oben beschrieben, ist es möglich, stereoselektive Reaktionen zu erzielen, so dass z. B. eine erleichterte Gewinnung von einzelnen Isomeren möglich ist.

Zu den Lösungsmitteln, aus denen die für die jeweilige Reaktion geeigneten ausgewählt werden können, zählen beispielsweise Wasser, Ester, wie Niederalkylniederalkanoate, z. B. Essigsäurediethylester, Ether, wie aliphatische Ether, z. B. Diethylether, oder cyclische Ether, z. B. Tetrahydrofuran, flüssige aromatische Kohlenwasserstoffe, wie Benzol oder Toluol, Alkohole, wie Methanol, Ethanol oder 1- oder 2-Propanol, Nitrile, wie Acetonitril, Halogenkohlenwasserstoffe, wie Methylenchlorid, Säureamide, wie Dimethylformamid, Basen, wie

heterocyclische Stickstoffbasen, z. B. Pyridin, Carbonsäureanhydride, wie Niederalkansäureanhydride, z. B. Acetanhydrid, cyclische, lineare oder verzweigte Kohlenwasserstoffe, wie Cyclohexan, Hexan oder Isopentan, oder Gemische dieser Lösungsmittel, z. B. wässrige Lösungen, soweit bei der Beschreibung der Verfahren nichts anderes angegeben ist. Derartige Lösungsmittelgemische können auch bei der Aufarbeitung, beispielsweise durch Chromatographie oder Verteilung, Verwendung finden.

Funktionelle Gruppen in Ausgangsmaterialien und den Zwischenprodukten, die nicht an der jeweiligen Reaktion teilnehmen sollen, insbesondere Aminogruppen (z.B. der Piperidinstickstoff oder der Aminooxystickstoff) oder Hydroxygruppen (wenn z. B. den Resten $W_1$ oder $W_2$ analoge Gruppen in Vorstufen zu Verbindungen der Formel V Hydroxy bedeuten), können durch geeignete Schutzgruppen (conventional protecting groups) geschützt sein, die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen. Als Schutzgruppen werden im Rahmen der vorliegenden Beschreibung allgemein nur solche Gruppierungen bezeichnet, die nicht in den Endprodukten vorliegen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate:Monosaccharide und Derivate", Georg Thieme Verlag, Stuttgart 1974, beschrieben.

Aminogruppen werden insbesondere geschützt, wie unter Verfahren (a) beschrieben. Dort finden sich auch bevorzugte Bedingungen zur Abspaltung der Aminoschutzgruppen.

Eine Hydroxygruppe kann beispielsweise durch eine Acylgruppe, z. B. durch Halogen, wie Chlor, substituiertes Niederalkanoyl, wie 2,2-Dichloracetyl, oder insbesondere durch einen für geschützte Aminogruppen genannten Acylrest eines Kohlensäurehalbesters oder unsubstituiertes oder substituiertes Benzoyl geschützt sein. Eine Hydroxygruppe kann auch durch Triniederalkylsilyl, z. B. Trimethylsilyl, Triisopropylsilyl oder tert-Butyl-di-methylsilyl, eine leicht abspaltbare aliphatische verethernde Gruppe, z. B. eine Alkylgruppe, wie tert-Niederalkyl, z. B. tert-Butyl, einen Oxa- oder einen thiaaliphatischen oder -cycloaliphatischen, insbesondere 2-Oxa- oder 2-thiaaliphatischen oder -cycloaliphatischen, Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, geschützt sein. Eine bevorzugte Hydroxyschutzgruppe ist beispielsweise 2,2,2-Trichlorethoxycarbonyl, 4-Nitrobenzyloxycarbonyl, 4-Nitrobenzoyl, Diphenylmethoxycarbonyl, Tetrahydropyran-2-yl oder Trityl.

Die Abspaltung der Schutzgruppen, die nicht Bestandteil des gewünschten Endproduktes der Formel I sind, z. B. der Amino- und/oder Hydroxyschutzgruppen, erfolgt in an sich bekannter Weise, z. B. mittels Solvolyse, insbesondere Hydrolyse, Alkoholyse oder Acidolyse, oder mittels Reduktion, insbesondere Hydrogenolyse oder chemischer Reduktion, sowie Photolyse, gegebenenfalls stufenweise oder gleichzeitig, wobei auch enzymatische Methoden verwendet werden können. Die Abspaltung der Schutzgruppen ist beispielsweise in den weiter vorn im Abschnitt über "Schutzgruppen" genannten Standardwerken beschrieben.

Eine durch eine geeignete Acylgruppe, eine Triniederalkylsilylgruppe oder durch gegebenenfalls substituiertes 1-Phenylniederalkyl geschützte Hydroxygruppe wird analog einer entsprechend geschützten Aminogruppe freigesetzt. Eine durch 2,2-Dichloracetyl geschützte Hydroxygruppe wird z. B. durch basische Hydrolyse, eine durch tert-Niederalkyl oder durch einen 2-Oxa- oder 2-thia-aliphatischen oder -cycloaliphatischen Kohlenwasserstoffrest geschützte Hydroxygruppe durch Acidolyse, z. B. durch Behandeln mit einer Mineralsäure oder einer starken Carbonsäure, z. B. Trifluoressigsäure, oder in Gegenwart eines Kationenaustauschers in der $H^+$-Form, eine durch unsubstituiertes oder substituiertes Benzoyl, z. B. 4-Nitrobenzoyl, geschützte Hydroxygruppe durch Alkoholyse, beispielsweise Methanolyse, insbesondere in Gegenwart einer katalytischen Menge eines Alkalimetallalkoholates, z. B. Natriummethylat (Natriummethanolat), freigesetzt.

In den Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt oder man das Verfahren auf irgendeiner Stufe abbricht, oder bei denen ein Ausgangsstoff unter den Reaktionsbedingungen gebildet oder in Form eines Derivates, z.B. eines Salzes davon, verwendet wird.

Salze von Zwischenverbindungen, die mindestens eine basische Gruppe aufweisen, beispielsweise entsprechende Verbindungen der Formel IV oder V, sind Säureadditionssalze, beispielsweise wie oben für Salze von Verbindungen der Formel I definiert. Bei Vorliegen von Schutzgruppen mit negativ geladenen Substituenten in den Zwischenverbindungen können auch Salze mit Basen gebildet werden, ferner Mischsalze oder innere Salze.

Neue Ausgangsstoffe und/oder Zwischenprodukte, insbesondere solche der Formel IV, sind ebenfalls Gegenstand der vorliegenden Erfindung. Vorzugsweise werden solche Ausgangsstoffe verwendet und die Reaktionsbedingungen so gewählt, dass man zu den in dieser Beschreibung als besonders bevorzugt aufgeführten Verbindungen gelangt.

Bevorzugte Zwischenverbindungen sind solche der oben gezeigten Formel IV, worin entweder $R_1$' ein Radikal der Formel IVa,

$$-(CH_2)_n-O-NX_2X_3 \qquad (IVa),$$

worin n 0 oder 1 ist und $R_2$' Wasserstoff bedeutet, oder $R_1$' Wasserstoff und $R_2$' ein Radikal der Formel IVb

$$-(CH_2)_p-O-NX_2X_3 \qquad (IVb),$$

worin p 1 oder 2 ist, bedeutet, und die übrigen Symbole die für Verbindungen der Formel I genannten Bedeutungen haben, wobei $X_1$, $X_2$ und $X_3$ unabhängig voneinander für eine Aminoschutzgruppe oder Wasserstoff stehen, mit der Massgabe, dass mindestens eine der Gruppen $X_1$, $X_2$ und $X_3$ eine Aminoschutzgruppe bedeutet, oder worin $X_1$ für eine Aminoschutzgruppe oder Wasserstoff steht und $X_2$ zusammen mit $X_3$ eine bivalente Aminoschutzgruppe bildet, oder Salze davon, sofern salzbildende Gruppen vorliegen.

Stärker bevorzugte Zwischenverbindungen der Formel IV sind solche, worin $X_1$ Wasserstoff oder eine Aminoschutzgruppe bedeutet, $R_1$' ein Radikal der in Anspruch 18 gezeigten Formel IVa bedeutet, worin n 0 ist und $X_2$ und $X_3$ Wasserstoff oder gemeinsam eine bivalente Aminoschutzgruppe bedeuten, $R_2$' Wasserstoff bedeutet, R $C_1$-$C_2$-Alkyl bedeutet und m 1 oder 2 ist, wobei R nur an solchen Ringkohlenstoff gebunden ist, der an das Stickstoffheteroatom des zentralen Piperidinringsystems direkt gebunden ist, oder Salze davon, sofern salzbildende Gruppen vorliegen.

In erster Linie bevorzugt ist eine Verbindung der Formel IV, worin $X_1$ Wasserstoff oder tert-Butoxycarbonyl bedeutet, $R_1$'-O-$NH_2$ oder -O-$NX_2$'$X_3$' bedeutet, worin $X_2$' und $X_3$' gemeinsam mit dem Stickstoff, an den sie gebunden sind, einen 1H-Isoindol-1.3(2H)-dionylrest bilden, R Methyl oder Ethyl bedeutet und m 1 oder 2 ist, oder Salze davon, sofern salzbildende Gruppen vorliegen; wobei in allererster Linie diejenigen Verbindungen bevorzugt sind, in denen der Rest R oder die Reste R in trans-Stellung zu $R_1$' stehen.

Die Erfindung betrifft insbesondere die in den Beispielen beschriebenen Verbindungen, neuen Zwischenverbindungen und Verfahren.

## Pharmazeutische Präparate

Die vorliegende Erfindung betrifft ebenfalls pharmazeutische Präparate, die als Wirkstoff eine der pharmakologisch wirksamen Verbindungen der Formel I oder ein pharmazeutisch annehmbares Salz davon enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, Alter, Gewicht, Hautfläche und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,01 g bis etwa 2 g, vorzugsweise von etwa 0,05 g bis etwa 1,0 g des Wirkstoffs, insbesondere von 0,1 bis 0,6 g.

Die vorliegende Erfindung betrifft ebenfalls die Verwendung von Verbindungen der Formel I (und gegebenenfalls der Formel IV als Pro-Drug) zur Herstellung von pharmazeutischen Präparaten zur Anwendung als ODC-Hemmer, beispielsweise zur Behandlung von Erkrankungen, die auf eine Hemmung der ODC ansprechen, insbesondere von den oben genannten Erkrankungen.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragées-Kerne können mit geeigneten, gegebenenfalls Magensaft-resistenten Ueberzügen versehen werden, wobei man u.a. konzentriene Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind beispielsweise in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände in Warmblütern, d.h. Säugetieren und insbesondere Menschen, vorzugsweise solchen Warmblütern, die einer derartigen Behandlung bedürfen. Die Verbindungen der Formel I der vorliegenden Erfindung oder ferner Pro-Drugs, insbesondere der Formel IV, und ihre pharmazeutischen Salze, sofern salzbildende Gruppen vorliegen, werden hierzu prophylaktisch oder therapeutisch verabreicht, wobei man sie vorzugsweise in Form von pharmazeutischen Präparaten verwendet, beispielsweise in einer zur Hemmung der Ornithindecarboxylase geeigneten, prophylaktisch oder therapeutisch gegen eine der genannten Krankheiten, z. B. Tumoren oder Protozoainfektionen, wirksamen Dosis. Dabei wird bei einem Körpergewicht von etwa 70 kg eine tägliche Dosis von etwa 0,3 g bis etwa 15 g, vorzugsweise von etwa 0,5 g bis etwa 5 g einer Verbindung der vorliegenden Erfindung verabreicht.

Die pharmazeutischen Präparate sind vorzugsweise solche, die geeignet sind, einem Warmblüter, z. B. Menschen, zur Therapie oder Prophylaxe einer der oben genannten Erkrankungen, die auf eine Hemmung der Ornithindecarboxylase anspricht, verabreicht zu werden und eine zur Hemmung dieses Enzyms wirksame

Menge einer Verbindung der Formel I oder ein pharmazeutisch annehmbares Salz hiervon, insbesondere zusammen mit wenigstens einem Trägermaterial, umfassen.

Die nachfolgenden Beispiele illustrieren die vorliegende Erfindung, ohne ihren Umfang einzuschränken; Temperaturen werden in Grad Celsius angegeben. Folgende Abkürzungen und Bezeichnungen werden verwendet: BOC ≙ tert-Butyloxycarbonyl; Essigester ≙ Essigsäureethylester, Ether ≙ Diethylether, $R_f$ ≙ Verhältnis von Laufstrecke zu Lösungsmittelfront bei Dünnschichtchromatographie; Smp. ≙ Schmelzpunkt; Sole ≙ gesättigte Kochsalzlösung; Zers. ≙ unter Zersetzung. Bei Lösungsmittel-, Verdünnungsmittel- und Elutionsmittelgemischen werden die Volumenanteile der Lösungsmittel am jeweiligen Gemisch (v:v) angegeben.

Beispiel 1: trans-2-Methyl-4-aminooxy-piperidin-sulfat

Ein Gemisch von 16,54 g (0,0472 Mol) 2-(trans-N-BOC-2-methyl-4-piperidyloxy)-1H-isoindol-1,3(2H)-dion in 45 ml Wasser und 30 ml konzentrierter Salzsäure wird unter Rühren 2 Stunden unter Rückfluss erhitzt, wobei zunächst eine Lösung entsteht, aus der aber nach kurzer Zeit wieder ein kristalliner Niederschlag (Phthalsäure) ausfällt. Das auf 0°C abgekühlte Reaktionsgemisch wird filtriert, der Filterrückstand mit Wasser gewaschen und das Filtrat im Vakuum eingedampft. Den Rückstand nimmt man in Ethanol auf und dampft im Vakuum zur Trockene ein. Nach nochmaligem Versetzen mit Ethanol und Eindampfen im Vakuum erhält man rohes trans-2-Methyl-4-aminooxy-piperidindihydrochlorid als harzartigen Rückstand. Der Rückstand wird in 30 ml Sole gelöst, von wenig ungelösten Bestandteilen abfiltriert und das Filtrat mit 20 ml 30 %iger Natronlauge basisch eingestellt. Anschliessend extrahiert man gründlich mit Methylenchlorid (2 Portionen à 100 ml und 2 Portionen à 50 ml), trocknet die vereinigte organische Phase über Natriumsulfat und dampft im Vakuum ein. Das als Öl erhaltene trans-2-Methyl-4-aminooxy-piperidin wird in 100 ml Ethanol gelöst und mit 42,6 ml 2N Schwefelsäure versetzt. Man filtriert dann das auskristallisierte Produkt ab, wäscht es mit Ethanol und Ether und trocknet bei 100° am Hochvakuum. Die erhaltene Titelverbindung schmilzt bei 251-253°C (Zers.).

Die Ausgangsverbindungen werden wie folgt hergestellt:

a) 2-(trans-N-BOC-2-methyl-4-piperidyloxy)-1H-isoindol-1,3(2H)-dion.

Zu einer Suspension von 25,2 g (0,117 Mol) cis-N-BOC-2-methyl-4-hydroxy-piperidin, 19,1 g (0,117 Mol) N-Hydroxyphthalimid und 30,7 g (0,117 Mol) Triphenylphosphin in 310 ml Benzol tropft man unter Rühren und Stickstoffatmosphäre bei 20-30°C eine Lösung von 19,68 g (0,1177 Mol) Azodicarbonsäure-diethylester (93 %) in 70 ml Benzol. Das Reaktionsgemisch wird 21/4 Stunden bei Raumtemperatur weitergerührt, auf 5°C abgekühlt und von ausgefallenem 1,2-Hydrazindicarbonsäure-diethylester abfiltriert. Nach Eindampfen des Filtrats im Vakuum löst man den Rückstand in 500 ml Ether und kühlt dann wieder auf 5°C ab. Ausgefallenes Triphenylphosphinoxid wird abfiltriert, das Filtrat im Vakuum eingedampft und der harzartige Rückstand mittels Flashchromatographie an Kieselgel der Korngrösse 0,04-0,063 mm unter Verwendung von Essigester-Hexan (3:1) gereinigt. Das noch leicht verunreinigte Produkt wird zur vollständigen Reinigung erneut über Kieselgel unter Verwendung von Essigester-Hexan-Gemischen (1:3 beziehungsweise 1:2 beziehungsweise 1:1) mittels Flashchromatographie aufgearbeitet. Nach Eindampfen der produkthaltigen Fraktionen erhält man die Titelverbindung als farbloses Harz, $R_f$-Wert = 0,65 (Kieselgel/Essigester:Hexan (2:1)).

b) cis-N-BOC-2-methyl-4-hydroxy-piperidin

Eine Lösung von 14,6 g (0,04876 Mol) cis-N-BOC-2-methyl-4-[(tetrahydropyran-2-yl)-oxy]-piperidin in 120 ml Methanol wird mit 20 g Kationenaustauscher Dowex® 50 WX8 ($H^+$-Form; 50 - 100 mesh (50 mesh enstspricht ca. 300 μm Korngrösse, 100 mesh ca. 150 μm); Kationenaustauscher auf Basis eines Styrol/Divinylbenzol-Polymeren mit Sulfonylgruppen; Warenzeichen der Dow Chemicals Co., USA)) versetzt und das Gemisch 22 Stunden bei Raumtemperatur gerührt. Nach Filtration, Waschen des Ionenaustauschers mit Methanol und Eindampfen des Filtrats am Vakuum erhält man die Titelverbindung in Form eines zähflüssigen Öls, $R_f$-Wert = 0,16 (Kieselgel/Essigester:Hexan (1:2)).

c) cis-N-BOC-2-methyl-4-[(tetrahydropyran-2-yl)oxy] -piperidin

Zu einer Lösung von 17,4 g (0,06 Mol) N-BOC-4-[(tetrahydropyran-2-yl)oxy]-piperidin in 120 ml Ether werden unter Stickstoffatmosphäre bei -65 bis -70°C 19,83 ml (0, 132 Mol) N,N,N'N'-Tetramethylethylendiamin und anschliessend 55,55 ml einer 1,3 molaren Lösung von sec-Butyllithium (0,0722 Mol) in Cyclohexan:Isopentan (92:8) getropft. Man rührt 3,5 Stunden bei -70°C und tropft dann im Verlauf von ca. 15 Minuten eine Lösung von 4,5 ml (0,07228 Mol) Methyliodid in 100 ml Ether zu, wobei die Innentemperatur auf maximal -60°C ansteigt. Man rührt 5 Minuten bei -60°C weiter, entfernt dann das Kältebad und lässt die Temperatur auf 20°C ansteigen. Das Reaktionsgemisch wird anschliessend unter Rühren tropfenweise mit 120 ml Wasser versetzt. Nach Abtrennen der organischen Phase extrahiert man die wässrige Phase noch dreimal mit je 75 ml Ether. Die vereinigten mit Sole gewaschenen, über Natriumsulfat getrockneten

Etherphasen werden im Vakuum eingedampft und der ölige Rückstand mittels Flashchromatographie an Kieselgel unter Verwendung von Essigester.Hexan (1:5) gereinigt. Nach Eindampfen der produkthaltigen Fraktionen erhält man die Titelverbindung als farbloses Öl, $R_f$-Wert = 0,49 (Kieselgel/Essigester:Hexan (1:2)).

d) N-BOC-4-[(tetrahydropyran-2-yl)oxy]-piperidin

Eine Lösung von 12,08 g (0,06 Mol) N-BOC-4-hydroxy-piperidin (vgl. EP 0 278 621) in 300 ml Methylenchlorid wird unter Rühren mit 8, 16 ml (0,09 Mol) 3,4-Dihydro-2H-pyran und 1,5 g (0,006 Mol) Pyridinium-(toluol-4-sulfonat) versetzt. Man rührt das Reaktionsgemisch 2,5 Stunden bei Raumtemperatur, wäscht es dann zweimal mit je 50 ml Sole: Wasser (1:1)-Gemisch, trocknet über Natriumsulfat und dampft im Vakuum ein, wobei man die Titelverbindung in Form eines farblosen Öls erhält, $R_f$-Wert = 0,58 (Kieselgel/Essigester:Hexan (2:1)), das bei 0°C allmählich kristallin erstarrt.

Beispiel 2: cis-2-Methyl-4-aminooxy-piperidin-sulfat

Ein Gemisch von 0,69 g (0,00197 Mol) 2-(cis-N-BOC-2-methyl-4-piperidyloxy)-1H-isoindol-1,3(2H)-dion in 3 ml Wasser und 2 ml konzentrierter Salzsäure wird unter Rühren 2,5 Stunden bei Rückflusstemperatur erhitzt und analog Beispiel 1 aufgearbeitet, wobei man die Titelverbindung erhält, Smp. 250-251 °C (Zers.).

Die Ausgangsverbindungen werden wie folgt hergestellt:

a) 2-(cis-N-BOC-2- methyl-4-piperidyloxy)-1H-isoindol-1,3(2H)-dion

Ein Gemisch von 0,65 g (0,00247 Mol) trans-N-BOC-2-methyl-4-hydroxy-piperidin, 0,403 g (0,00247 Mol) N-Hydroxy-phthalimid und 0,648 g (0,00247 Mol) Triphenylphosphin in 10 ml Benzol wird analog Beispiel 1a mit einer Lösung von 0,434 ml (0,00256 Mol) Azodicarbonsäure-diethylester (93 %) in 2 ml Benzol umgesetzt. Nach Abfiltrieren von 1,2-Hydrazindicarbonsäure-diethylester wird das Filtrat im Vakuum eingedampft und der ölige Rückstand mittels Flashchromatographie an Kieselgel unter Verwendung von Essigester:Hexan (1:3) gereinigt. Man erhält die Titelverbindung als farbloses Harz, $R_f$-Wert = 0,53 (Kieselgel/Essigester:Hexan (1:1)).

b) trans-N-BOC-2-methyl-4-hydroxy-piperidin Ein Gemisch von 0,9 g (0,00246 Mol) trans-N-BOC-2-methyl-4-(4-nitro- benzoyloxy)piperidin, 12 ml Methanol und 0,022 ml (0,000119 Mol) einer 30 %igen Lösung von Natriummethylat in Methanol wird 15 Stunden bei Raumtemperatur gerührt. Man filtriert, dampft das Filtrat im Vakuum ein und reinigt den Rückstand mittels Flashchromatographie an Kieselgel unter Verwendung von Essigester:Hexan-Gemischen (1:2 beziehungsweise 1:1). Nach Eindampfen der produkthaltigen Fraktionen erhält man die Titelverbindung als farbloses Öl, $R_f$-Wert = 0,59 (Kieselgel/Essigester:Hexan (1:2)).

c) trans-N-BOC-2-methyl-4-(4-nitro-benzoyloxy)-piperidin

Zu einer Suspension von 2,15 g (0,01 Mol) cis-N-BOC-2-methyl-4-hydroxy-piperidin (vgl. Beispiel 1b), 2,0 g (0,012 Mol) 4-Nitro-benzoesäure und 3,15 g (0,012 Mol) Triphenylphosphin in 30 ml Toluol tropft man bei 5-10°C unter Rühren eine Lösung von 2 ml (0,012 Mol) Azodicarbonsäure-diethylester (93 %) in 10 ml Toluol. Das Reaktionsgemisch wird 15 Stunden bei Raumtemperatur weitergerührt und anschliessend von ausgefallenem Hydrazindiacarbonsäure-diethylester abfiltriert. Man dampft dann das Filtrat im Vakuum ein und reinigt den öligen Rückstand mittels Flashchromatographie an Kieselgel unter Verwendung von Essigester:Hexan (1:5). Nach Eindampfen der produkthaltigen Fraktionen erhält man die Titelverbindung als kristallinen Rückstand, Smp. 106-108°C.

Beispiel 3: trans-2-Ethyl-4-aminooxy-piperidin-dihydrochlorid

Ein Gemisch von 0,217 g (0,0005795 Mol) 2-(trans-N-BOC-2-ethyl-4-piperidyloxy)-1H-isoindol-1,3(2H)-dion, 3 ml Wasser und 2 ml konzentrierter Salzsäure wird analog Beispiel 1 umgesetzt. Beim Eindampfen mit Ethanol fällt die Titelverbindung in kristalliner Form an. Nach Umkristallisation aus Ethanol/Ether schmilzt sie bei 206-207°C (Zers.).

Die Ausgangsverbindungen werden wie folgt hergestellt:

a) 2-(trans-N-BOC-2-ethyl-4-piperidyloxy)-1H-isoindol-1,3(2H)-dion

Zu einer Suspension von 0,37 g (0,001613 Mol) cis-N-BOC-2-ethyl-4-hydroxy-piperidin, 0,263 g (0,001613 Mol) N-Hydroxy-phthalimid und 0,423 g (0,001613 Mol) Triphenylphosphin in 5 ml Benzol tropft man unter Rühren und Stickstoffatmosphäre bei 20-30°C eine Lösung von 0,283 ml (0,001693 Mol) Azodicarbonsäure-diethylester (93 %) in 1 ml Benzol. Das Reakionsgemisch wird 15 Stunden bei Raumtemperatur weitergerührt, von ausgefallenem 1,2-Hydrazindicarbonsäure-diethylester abfiltriert und das Filtrat im Vakuum eingedampft. Nach Reinigung des Rückstands mittels Flashchromatographie an Kieselgel unter Verwendung von Essigester:Hexan (1:3) erhält man die Titelverbindung als farbloses Harz, $R_f$-Wert = 0,66 (Kieselgel/Essigester:Hexan (2:1)).

b) cis-N-BOC-2-ethyl-4-hydroxy-piperidin und trans-N-BOC-2-ethyl-4-hydroxy-piperidin Ein Gemisch von 2,5 g (0,00798 Mol) N-BOC-2-ethyl-4-[(tetrahydropyran-2-yl)oxy]-piperidin (cis/trans-Gemisch), 25 ml Methanol und 3,5 g Kationenaustauscher Dowex® 50 WX8 ($H^+$-Form; 50-100 mesh (50 mesh enstspricht ca. 300 µm Korngrösse, 100 mesh ca. 150 µm); Kationenaustauscher auf Basis eines Styrol/Divinylbenzol-Polymeren mit Sulfonylgruppen; Warenzeichen der Dow Chemicals Co., USA) wird 15 Stunden bei Raumtemperatur gerührt. Nach Filtration, Waschen des Ionenaustauschers mit Methanol und Eindampfen des Filtrats am Vakuum reinigt man den öligen Rückstand mittels Flashchromatographie an Kieselgel unter Verwendung von Essigester:Hexan-Gemischen (1:3 beziehungsweise 1:2). Die produkthaltigen Fraktionen werden eingedampft, wobei man die cis-Titelverbindung, $R_f$-Wert = 0,17 und die trans-Titelverbindung $R_f$-Wert = 0,12 (Kieselgel/Essigester:Hexan (1:2)) jeweils als Öl erhält.

c) N-BOC-2-ethyl-4-[(tetrahydropyran-2-yl)oxy]-piperidin Analog Beispiel 1c wird eine Lösung von 8,75 g (0,03 Mol) N-BOC-4-[tetrahydropyran-2-yl)oxy]-piperidin (vgl. Beispiel 1d) in 60 ml Ether mit 9,92 ml (0,066 Mol) N,N,N',N'-Tetramethylethylendiamin und 27,78 ml (0,0361 Mol) einer 1,3-molaren Lösung von sec-Butyllithium in Cyclohexan:Isopentan (92:8) und einer Lösung von 2,92 ml (0,03614 Mol) Ethyliodid in 50 ml Ether umgesetzt. Nach Reinigung des Rohprodukts mittels Flashchromatographie an Kieselgel unter Verwendung von Essigester:Hexan-Gemischen (1:6 beziehungsweise 1:5) erhält man die als cis/trans-Gemisch vorliegende Titelverbindung in Form eines farblosen Öls, $R_f$-Wert = 0,39 (Kieselgel/Essigester:Hexan (1:3)).

Beispiel 4: cis-2-Ethyl-4-aminooxy-piperidin-sulfat

Ein Gemisch von 0,85 g (0,00227 Mol) 2-(cis-N-BOC-2-ethyl-4-piperidyloxy)-1H-isoindol-1,3(2H)-dion, 6 ml Wasser und 4 ml konzentrierter Salzsäure wird analog Beispiel 1 umgesetzt. Das rohe, harzartige Dihydrochlorid der Titelverbindung führt man, wie in Beispiel 1 beschrieben, in das kristalline Sulfat über, Smp. 234-235°C (Zers.).

Die Ausgangsverbindung wird wie folgt hergestellt:

a) 2-(cis-N-BOC-2-ethyl-4-piperidyloxy)-1H-isoindol-1,3(2H)-dion

Eine Suspension von 0,74 g (0,003227 Mol) trans-N-BOC-2-ethyl-4-hydroxy-piperidin (vgl. Beispiel 3b), 0,526 g (0,003227 Mol) N-Hydroxy-phthalimid und 0,846 g (0,003227 Mol) Triphenylphosphin in 10 ml Benzol wird analog Beispiel 3a mit einer Lösung von 0,566 ml (0,003385 Mol) Azodicarbonsäure-diethylester (93 %) in 2 ml Benzol umgesetzt. Man erhält die Titelverbindung als farbloses Harz, $R_f$-Wert = 0,60 (Kieselgel/Essigester:Hexan (2:1)).

Beispiel 5: 2t,6t-Dimethyl-4r-aminooxypiperidin-dihydrochlorid

Ein Gemisch von 2,0 g (0,00534 Mol) 2-[N-BOC-(2t,6t-dimethyl)-4r-piperidyloxy]-1H-isoindol-1,3(2H)-dion, 10 ml Wasser und 8,5 ml konzentrieter Salzsäure wird analog Beispiel 1 umgesetzt. Zur Reinigung kristallisiert man das rohe Dihydrochlorid der Titelverbindung aus Methanol/Ether um, Smp. 211°C (Zers.).

Die Ausgangsverbindungen werden wie folgt hergestellt:

a) 2-[N-BOC-(2t,6t-dimethyl-4r-piperidyloxy]-1H-isoindol-1,3(2H)-dion

Eine Suspension von 6,0 g (0,02616 Mol) N-BOC-2c,6c-dimethyl-4r-hydroxy-piperidin, 4,27 g (0,02616 Mol) N-Hydroxy-phthalimid und 6,86 g (0,02616 Mol) Triphenylphosphin in 50 ml Benzol wird analog Beispiel 3a mit einer Lösung von 4,6 ml (0,0275 Mol) Azodicarbonsäure-diethylester (93 %) in 15 ml Benzol umgesetzt. Nach Reinigung des Rohprodukts mittels Flashchromatographie an Kieselgel unter Verwendung von Essigester:Hexan-Gemischen (1:4 beziehungsweise 1:2) erhält man die Titelverbindung als farbloses Harz, das allmählich kristallin erstarrt, Smp. 107-109°C.

b) N-BOC-2c,6c-dimethyl-4r-hydroxy-piperidin

Ein Gemisch von 3,6 g (0,02786 Mol) 2c,6c-dimethyl-4r-hydroxy-piperidin [vgl. J. Org. Chem. 15, 337-342 (1950) und Beilstein, 21, EIII/IV, 112], 6,7 g (0,03069 Mol) Di-tert-butyl-dicarbonat und 40 ml Methylenchlorid wird 24 Stunden unter Rückfluss erhitzt und anschliessend 65 Stunden bei 20°C stehen gelassen. Man fügt eine zweite Portion von 6,7 g (0,03069 Mol) Di-tert-butyl-dicarbonat zum Reaktionsgemisch und erhitzt nochmals 24 Stunden unter Rückfluss. Das Reaktionsgemisch wird dann im Vakuum eingedampft und der ölige Rückstand mittels Flashchromatographie an Kieselgel unter Verwendung von Essigester:Hexan-Gemischen (1:4 beziehungsweise 1:1) gereinigt. Nach Eindampfen der produkthaltigen Fraktionen erhält man die Titelverbindung als farbloses Öl, $R_f$-Wert = 0,32 (Kieselgel/Essigester:Hexan (1:1)).

Beispiel 6: Kapseln

Kapseln enthaltend 0,25 g Wirkstoff, z.B. eine der Verbindungen der Beispiele 1-5, können wie folgt hergestellt werden:

| Zusammensetzung (für 5000 Kapseln) | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

**Patentansprüche**

1. Verbindungen der Formel I,

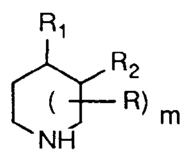

(I)

worin entweder $R_1$ ein Radikal der Formel Ia,

$$-(CH_2)_n-O-NH_2 \quad (Ia)$$

in dem n 0 oder 1 ist, und $R_2$ Wasserstoff bedeuten, oder $R_1$ Wasserstoff und $R_2$ ein Radikal der Formel Ib,

$$-(CH_2)_p-O-NH_2 \quad (Ib)$$

in dem p 1 oder 2 ist, bedeuten; und worin R $C_1$-$C_2$-Alkyl bedeutet, welches an ein Kohlenstoffatom des zentralen Piperidinringsystems gebunden ist, aber nicht an dasselbe Kohlenstoffatom wie $R_1$ der Formel Ia oder wie $R_2$ der Formel Ib; wobei m 1 oder 2 bedeutet, und Salze davon.

2. Eine Verbindung gemäss Anspruch 1 der Formel II,

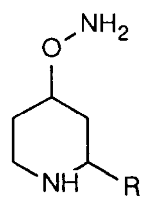

(II)

worin R $C_1$-$C_2$-Alkyl bedeutet, und pharmazeutisch annehmbare Salze davon.

3. Eine Verbindung gemäss Anspruch 5 der Formel II, worin der Rest $-O-NH_2$ und der Rest R, welcher $C_1$-$C_2$-Alkyl bedeutet, in trans-Stellung zueinander an das zentrale Piperidinringsystem gebunden sind, und pharmazeutisch annehmbare Salze davon.

4. Eine Verbindung gemäss Anspruch 1 der Formel III

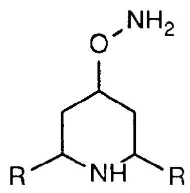

(III)

worin die Reste R unabhängig voneinander $C_1$-$C_2$-Alkyl bedeuten, und pharmazeutisch annehmbare Salze davon.

5. Eine Verbindungen der Formel I gemäss Anspruch 1 mit dem Namen trans-2-Methyl-4-aminooxy-piperidin, oder pharmazeutisch annehmbare Salze davon.

6. Eine Verbindung der Formel I oder ein Salz davon gemäss einem der Ansprüche 1 bis 5 zur Anwendung in einem Verfahren zur therapeutischen Behandlung des menschlichen oder tierischen Körpers.

7. Pharmazeutisches Präparat enthaltend eine Verbindung der Formel I oder ein Salz davon gemäss einem der Ansprüche 1 bis 5, oder ein pharmazeutisch annehmbares Salz davon, und wenigstens ein pharmazeutisch verwendbares Trägermaterial.

8. Verwendung einer der in in den Ansprüchen 1 bis 5 genannten Verbindungen der Formel I, oder eines pharmazeutisch verwendbaren Salzes davon, zur Herstellung von pharmazeutischen Präparaten zur Anwendung zur Behandlung von Erkrankungen, die auf eine Hemmung der Ornithindecarboxylase ansprechen.

9. Verfahren zur Herstellung von Verbindungen der Formel I gemäss Anspruch 1, dadurch gekennzeichnet, dass man
(a) aus einer Verbindung der Formel I, worin mindestens eine Aminogruppe geschützt ist, die Aminoschutzgruppe(n) abspaltet, oder
(b) eine Verbindung der Formel V

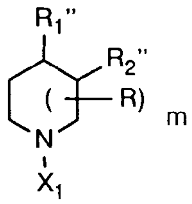

(V)

oder einem Salz davon, sofern salzbildende Gruppen vorliegen, worin $X_1$ Wasserstoff oder eine Aminoschutzgruppe bedeutet; entweder $R_1$" einen Rest der Formel Va,

$-(CH_2)_n-W_1$ (Va)

worin $W_1$ eine Abgangsgruppe bedeutet und n 0 oder 1 ist, und $R_2$" Wasserstoff bedeuten; oder $R_1$" Wasserstoff und $R_2$" einen Rest der Formel Vb

$-(CH_2)_p-W_2$ (Vb)

bedeuten, worin $W_2$ eine Abgangsgruppe bedeutet und p 1 oder 2 ist; und die übrigen Symbole die für Verbindungen der Formel I genannten Bedeutungen haben; mit einem aminogeschützten Hydroxylamin-Derivat unter Substitution entweder von $W_1$ oder $W_2$ umsetzt, wobei weitere funktionelle Gruppen in den Ausgangsmaterialien, die nicht an der Reaktion teilnehmen sollen, in geschützter Form vorliegen, und vorhandene Schutzgruppen abspaltet,
und gewünschtenfalls eine Verbindung der Formel I in eine andere Verbindung der Formel I überführt, ein erhältliches Isomerengemisch in die Isomeren aufspaltet und/oder eine erhältliche freie Verbindung der Formel I in ein Salz überführt oder ein erhältliches Salz in die freie Verbindung der Formel I überführt oder in ein anderes Salz umwandelt.

10. Verbindungen der Formel IV,

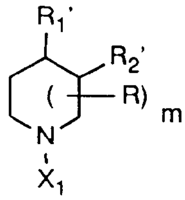

worin entweder $R_1'$ ein Radikal der Formel IVa,

$$-(CH_2)_n-O-NX_2X_3 \quad (IVa),$$

worin n 0 oder 1 ist, und $R_2'$ Wasserstoff bedeutet, oder $R_1'$ Wasserstoff und $R_2'$ ein Radikal der Formel IVb

$$-(CH_2)_p-O-NX_2X_3 \quad (IVb),$$

worin p 1 oder 2 ist, bedeutet, und die übrigen Symbole die in Anspruch 1 für Verbindungen der Formel I genannten Bedeutungen haben, wobei $X_1$, $X_2$ und $X_3$ unabhängig voneinander für eine Aminoschützgruppe oder Wasserstoff stehen, mit der Massgabe, dass mindestens eine der Gruppen $X_1$, $X_2$ und $X_3$ eine Aminoschutzgruppe bedeutet, oder worin $X_1$ für eine Aminoschutzgruppe oder Wasserstoff steht und $X_2$ zusammen mit $X_3$ eine bivalente Aminoschutzgruppe bildet, oder Salze davon.

Europäisches Patentamt

# EUROPÄISCHER RECHERCHENBERICHT

Nummer der Anmeldung

EP 93 81 0079

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| P,X | EP-A-0 495 750 (CIBA-GEIGY A.-G.; SWITZ. (CH))<br>22. Juli 1992<br>* das ganze Dokument * | 1-10 | C07D211/22<br>C07D211/42<br>C07D211/46<br>A61K31/445 |
| A | EP-A-0 452 264 (CIBA-GEIGY)<br>16. Oktober 1991<br>* das ganze Dokument * | 1-10 | |
| A | EP-A-0 369 944 (CIBA-GEIGY)<br>23. Mai 1990<br>* das ganze Dokument * | 1-10 | |
| A | CHEMICAL ABSTRACTS, vol. 104, 1986, Columbus, Ohio, US;<br>abstract no. 30840,<br>KHOMUTOV ET. AL. 'Aminooxypropylamine as an effective inhibitor of ornithine decarboxylase in vitro and vivo.'<br>* Zusammenfassung *<br>& BIOORG. KHIM.<br>Bd. 11, Nr. 11, 1985,<br>Seiten 1574 - 1576 | 1-10 | |

| RECHERCHIERTE SACHGEBIETE (Int. Cl.5 ) |
|---|
| C07D<br>C07C |

Der vorliegende Recherchenbericht wurde für alle Patentansprüche erstellt

| Recherchenort | Abschlußdatum der Recherche | Prüfer |
|---|---|---|
| DEN HAAG | 19 MAERZ 1993 | Bernd Kissler |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)